# EUROPEAN PATENT APPLICATION

(11) **EP 0 803 602 A1**
(43) Date of publication of application: **29.10.1997**
(21) Application number: 96900439.9
(22) Date of filing: 12.01.1996
(51) Int. Cl.: D04H 3/16, A61F 13/56, A61F 13/15, B32B 5/04, B32B 5/26, B32B 25/10, B32B 27/12

(54) **COMPOSITE ELASTIC BODY HAVING MULTISTAGE ELONGATION CHARACTERISTICS AND METHOD OF MANUFACTURING THE SAME**

(30) Priority: 12.01.1995 JP 2973/95; 18.08.1995 JP 210600/95
(71) Applicant: JAPAN ABSORBENT TECHNOLOGY INSTITUTE, Chuo-ku, Tokyo 103 (JP)
(72) Inventor: SUZUKI, Migaku, Kamakura-shi, Kanagawa 247 (JP); FUKUI, Hiroaki, Kawaguchi-shi, Saitama 332 (JP)
(74) Representative: Klingseisen, Franz, Dipl.-Ing.
(86) International application number: PCT/JP96/00041
(87) International publication number: WO 96/21760

(57) **Abstract**

There is provided a composite elastic material comprising a non-woven fabric having an elongation along at least one direction and a break-down elasticity along said direction of 100% or more and a sheet-like elastic member having an elastic recovery of 60% or more and a break-down elasticity of 200% or more, with the elastic member and the non-woven fabric being secured together at a plurality of securements discontinuous in the stretchable direction of the non-woven fabric, and the method of producing the same. The composite elastic material provides multiple-stage elongation characteristic having a first stress lowering point caused by changes of structure of said non-woven fabric while being stretched along said direction, and a second stress lowering point occurring at an elongation larger than that said first stress lowering point lies, caused by break-down of said sheet-like elastic member. Therefore, the elastic material shows clearly a break-down point when stretched, and increases flexibility on designing various products.

## Description

### Field of the Invention

The present invention relates to a composite elastic material comprising a non-woven fabric and an elastic sheet, which has different stretch-recovery properties according to the degree of elongation. This composite elastic material can be advantageously used for the purposes of an elastic material applied to parts which directly touch the skin of the human body, such as an elastic material which is provided for the lumbar region or the crotch region of sanitary articles such as baby and adult diapers, and the sleeve portion of a medical gown.

### Prior Art

Recently, the use of an elastic member is expanded into disposal goods such as medical articles and hygienic articles in order to improve the fitness for the human body. In particular, for baby articles and the like, an elastic material is scarcely used in its original form, but almost used in the form of a composite with a non-woven fabric. In a composite of the non-woven fabric and the elastic sheet, elastic functions are demanded from the elastic sheet; and the functions such as an improvement of the surface condition of the composite, and the effect of reinforcing the elastic sheet are demanded from the non-woven fabric.

Typical examples of such composite elastic material include a three-layer composite elastic material which is referred to as S.M.S. (spun-bond/melt-blown/spun-bond) made by Kimbarry, U.S.A., as disclosed in JPA Nos.84143/87, 28456/87 and 33889/87. Such composite elastic material is manufactured according to a system which is referred to as S.B.L. (Stretched Bonding Laminate), which comprises stretching an elastic sheet to laminate the sheet to a non-woven fabric in an elongated state, and thereafter loosening the laminate. According to the system, since the stretch range is stable, in particular, the critical value of elongation corresponds to the elongation range on manufacturing, the composite elastic material will be neither elongated beyond that in a usual application state nor fractured. However, the system has many inconvenient aspects for a commercial production at high velocity on the points that it is necessary to use the non-woven fabric is used in an amount more than a needed amount; and that the completed article is of great bulk.

JPA No.281059/92 discloses a system in which a fiber is directly entangled with an elastic net, but the system has a defect in manufacturing costs. In order to dissolve the defect, there has been an attempt using an elongating non-woven fabric so as to provide a composite elastic material having a structure in channel state, in which the non-woven fabric is line-bonded to an elastic film has been also made (JPA No.222601/93).

A prior composite elastic material as mentioned above is capable of being elongated in a large range. However, it is indefinite where the limiting point exists, which is a critical problem to design goods. Furthermore, from an aspect of utilization thereof, there is left the sense of uneasiness that it is unclear that the composite elastic material is broken by any long elongation on wearing.

### Summary of the Invention

It is an object of the present invention to provide a composite elastic material which has the inherent properties of a combined non-woven fabric and elastic sheet, and is excellent in the productivity and the economics, and also in the function, and also to provide a method of manufacturing such composite elastic material.

Another object of the present invention is to provide an absorbent product which has the above-mentioned composite elastic material as a component.

According to the present invention, there is provided a composite elastic material having a multiple-stage elongation property comprising:
a non-woven fabric which is stretchable along at least one direction and has a break-down elasticity along said direction of 100% or more; and
a sheet-like elastic member having an elastic recovery of 60% or more and a break-down elasticity of 200% or more;
said non-woven fabric and said elastic member being secured together at a plurality of securements, so that said composite elastic material has a first stress lowering point caused by changes of structure of said non-woven fabric while being stretched along said direction, and a second stress lowering point occurring at an elongation larger than that said first stress lowering point lies, caused by break-down of said sheet-like elastic member.

Namely, the feature of a composite elastic material having an elongation activity, of the present invention is that the composite elastic material is elongated with an approximate constant increase of stress until the composite elastic material reaches a first elongation point in the step in which the composite elastic material is uniaxially elongated in a predetermined direction, but when the composite elastic material is further elongated after the stress has reached a maximum at the first inflection point, the stress is rapidly lowered until a certain value, and thereafter, when the composite elastic material is further continuously elongated, it is elongated with a low level stress, and when it has reached a second inflection point, it is fractured.

A non-woven fabric which is one of the constituents of the composite elastic material of the present invention as mentioned above is an easily stretchable non-woven fabric which was provided according to a method which is characterized in that a composite non-woven fabric is heated to draw at a temperature of the softening point or more of a jointing component (A) which is one of the constituents of the composite non-woven fabric and has an easily thermoplastic property, and at a temperature in the range of stable temperatures of a skeleton component (B); the composite non-woven fabric comprising a fibrous material which comprises the jointing component (A) which has an easily thermoplastic property, and the skeleton component (B) which has a relatively high heat-stability. Since the composite non-woven fabric has an elongating property of 100% or more in one direction, and is excellent in the aspect of a feel such as the touch, the composite elastic material which is provided in combination with the composite non-woven fabric is quite suitable for an elastic material of a product such as an underwear or a sanitary article.

In the present invention, in order to provide a structure having an easily elongating property with maintaining the morphology and the properties of a non-woven fabric, it is necessary to reorient constitutive fibers with maintaining the warp-weft bond thereof. In order to satisfy this condition, the present invention adopts a means in which the jointing component (A) of the composite non-woven fabric which comprises the jointing component(A) and the skeleton component (B) as mentioned above is heated to plasticize it, and is reoriented, while the space between the constitutive fibers sliding through a drawing operation by using the fluidity of the jointing component (A) caused flowable.

### Brief Description of the Drawings

Figure 1 shows S-S curves obtained from a composite elastic material of the present invention, a non-woven fabric and an elastic sheet, and the non-woven fabric and the elastic sheet constituting the composite elastic material, in the state that each was elongated until being broken;
Figure 2 shows an S-S curve obtained from the composite elastic material of the present invention which was not predrawn, when it was elongated until being broken;
Figure 3 shows an S-S curve obtained from a sample of a composite elastic material of the present invention which was predrawn to 75%, when the sample was elongated from no drawn state until being broken;
Figure 4 shows an S-S curve obtained from a sample of a composite elastic material of the present invention which was predrawn to 100%, when the sample was elongated from no drawn state until being broken;
Figure 5 shows an S-S curve obtained from a sample of a composite elastic material of the present invention which was predrawn to 150%, when the sample was elongated from no drawn state until being broken;
Figure 6 is a graph showing four different areas of the elastic property, which were formed in an S-S curve of a sample of a composite elastic material of the present invention which was predrawn to 150%, when the sample was elongated from no drawn state until being broken;
Figure 7 shows an S-S curve obtained from a composite elastic material of the present invention, when it was elongated to 150%, and thereafter recovered;
Figure 8 shows an S-S curve obtained from another composite elastic material of the present invention, when it was elongated to 150%, and thereafter recovered;
Figure 9 shows an S-S curve obtained from a composite elastic material of the present invention, when it was elongated to 50%, and thereafter recovered;
Figure 10 shows an S-S curve obtained from a composite elastic material of the present invention, when it was elongated to 75%, and thereafter recovered;
Figure 11 shows an S-S curve obtained from a composite elastic material of the present invention, when it was elongated to 100%, and thereafter recovered;
Figure 12 shows an S-S curve obtained from a composite elastic material of the present invention, when it was elongated to 120%, and thereafter recovered;
Figure 13 shows an S-S curve obtained from a composite elastic material of the present invention, when it was elongated to 150%, and thereafter recovered;
Figure 14 shows an S-S curve obtained from a non-woven fabric which is provided by a water entangling, in MD and CD, which is used in the present invention;
Figure 15 is a schematic diagram showing an example of processes for manufacturing an easily elongating non-woven fabric according to the present invention;
Figure 16 is a sectional view showing an example of the construction of a composite non-woven fabric which is suitable for the use as a fabric material for a composite elastic material of the present invention;
Figure 17 is a sectional view showing another example of the constructions of a composite non-woven fabric which is suitable for the use as a fabric material for a composite elastic material of the present invention;
Figure 18 is a plan view showing the bonding pattern of the composite non-woven fabric which is suitable for the use as a fabric material for the composite elastic material of the present invention;
Figure 19 is a schematic sectional view showing a first example of the arrangement of the elastic material sheet and the non-woven fabric for the present invention;
Figure 20 is a schematic sectional view showing a second example of the arrangement of the elastic material sheet and the non-woven fabric for the present invention;
Figure 21 is a schematic sectional view showing a third example of the arrangement of the elastic material sheet and the non-woven fabric for the present invention;
Figure 22 is an explanation drawing showing an example of the process for manufacturing the composite elastic material of the present invention;
Figure 23 is a schematic diagram showing another example of the process for manufacturing the composite elastic material of the present invention;
Figure 24 is a plan view showing a first example of the pattern of discontinuous bond sites which are applied to the composite elastic material of the present invention;
Figure 25 is a plan view showing a second example of the pattern of discontinuous bond sites which are applied to the composite elastic material of the present invention;
Figure 26 is a plan view showing a third example of the pattern of discontinuous bond sites which are applied to the composite elastic material of the present invention;
Figure 27 is a plan view showing a fourth example of the pattern of discontinuous bond sites which are applied to the composite elastic material of the present invention;
Figure 28 is a plan view showing a fifth example of the pattern of discontinuous bond sites which are applied to the composite elastic material of the present invention;
Figure 29 is a plan view showing the direction of elongation of the composite elastic material of the present invention;
Figure 30 is a plan view showing a first example of the configuration of discontinuous bond sites which are applied to the composite elastic material of the present invention;
Figure 31 is a plan view showing a second example of the configuration of discontinuous bond sites which are applied to the composite elastic material of the present invention;
Figure 32 is a plan view showing one example of the configuration of discontinuous bond sites which are applied to the composite elastic material of the present invention;
Figure 33 is a plan view showing another example of the configuration of discontinuous bond sites which are applied to the composite elastic material of the present invention;
Figure 34 is a plan view showing further another example of the configuration of discontinuous bond sites which are applied to the composite elastic material of the present invention;
Figure 35 is a graph showing the results obtained from the measurement of the relationship between a temperature at the press under heat and a tensile strength of the composite elastic material of the present invention;
Figure 36 is a perspective view showing an absorbent product in which the composite elastic material shown in Figure 13 is used as a side panel;
Figure 37 is a perspective view showing an absorbent product in which the composite elastic material shown in Figure 13 is used as a side panel;
Figure 38 is S-S curves of a composite elastic material, an elastic material sheet and a non-woven fabric in the CD; the composite elastic material being provided in Example 1 of the present invention, the elastic material sheet and the non-woven fabric being used for the composite elastic material;
Figure 39 shows an example of the process for drawing a non-woven fabric which is used for a composite elastic material of the present invention; and
Figure 40 is a schematic diagram showing another example of the process for manufacturing a composite elastic material having elastic properties in both warp and weft directions according to the present invention.

### Detailed Description of the Invention

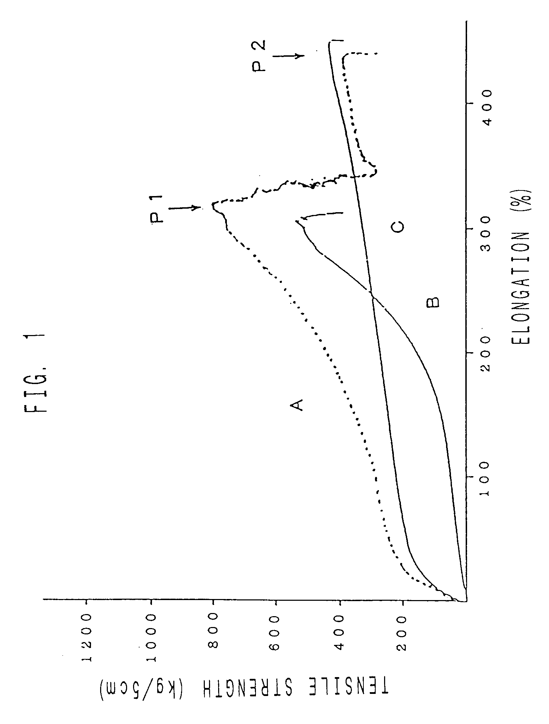

As a typical embodiment, a composite elastic material will be described with reference to Figure 1.

The composite elastic material is constituted by jointing the following non-woven fabric and elastic material sheet with dots which are discontinuous with each other in an approximately perpendicular direction to the elongating direction thereof.
Nonwoven Fabric: A water entangling web having a machine/cross elongation ratio of 1/4 and a weight of 20g/cm², which is a polyester fiber obtained according to the card process; and
Elastic Member: A film having a thickness of 40µm, which is obtained by die-extruding a compound comprising 80 parts of S.E.B.S. resin and 20 parts of E.V.A.

Curve(A) in Figure 1 shows the relationship (S-S curves) between an elongation (strain) and a stress of the composite elastic material, when the composite elastic material was elongated in an easily stretchable direction (CD) at a constant speed. Curve (B) shows an S-S curve of the non-woven fabric alone, while a curve (C) shows the one of the elastic material sheet alone.

As increase in the elongation degree of the composite elastic material, elongation will occur rapidly at first, and then slowly, thereafter, while the increase rate of the stress again becomes high as it approaches the breaking point of the non-woven fabric, and the stress is rapidly lowered when it has reached the breaking point of the non-woven fabric. This first inflection point (P1) is the breaking point of the non-woven fabric, and the elongation thereafter is carried on with a stress which approximates the S-S curve of the elastic sheet itself, and then the composite elastic material is completely fractured when the stress has reached the second inflection point (P2).

That is, the composite elastic material of the present invention has two stress-lowering points (P1) and (P2) in process of being elongated in a predetermined direction. The first stress-lowering point (P1) is provided when the non-woven fabric is elongated over the elongation limit thereof, while the second stress-lowering point (P2) is provided by the breaking of the elastic sheet.

The composite elastic material having multistage elongation properties as mentioned above can be used for various purposes, in particular, as a component of parts which touch the skin, of medical or sanitary articles. As an example, a material for the waist gather of a diaper can be cited. The waist gather of a diaper will have an excellent elastic property since it is largely expanded on putting on and off, and furthermore can be avoid the trouble that the waist gather is in error ripped up by perceiving that it is impossible to further elongate the waist gather before it has reached the breaking limit thereof and has been fractured. According to a composite elastic material of the present invention, since the stress will be increased as the elongation percentage is increased, as an elongation property of a composite elastic material which has a common non-woven fabric, the elongation limit thereof can be taken in. Moreover, when the composite elastic material was in error subjected to a further elongation, the non-woven fabric is fractured, and the whole stress is rapidly lowered, but since the elastic sheet is not fractured, a further elongation will be allowed for the elastic property until the elastic sheet has reached the strength against breaking.

A non-woven fabric which can be applied to a composite elastic material of the present invention has an elongation property in the direction at least one axis, and has an elongation percentage of 100% or more, preferably 150% or more in this elongating direction. If the elongation percentage is less than 100%, the range of uses as an elastic material is sharply restricted, and the utility thereof is lowered.

It is necessary that the elastic sheet has an elastic recovery percentage of 60% or more and an elongation percentage after breaking of 200% or more, preferably 250% or more. If the elastic recovery percentage is less than 60% and/or the elongation percentage after breaking is less than 200%, an elastic property which needs to use the elastic sheet for the purposes as mentioned above is not provided.

Furthermore, it is preferable that the difference between the elongation percentage after breaking of the non-woven fabric and that of the elastic sheet is as large as possible, and if it is 100% or more, they can be advantageously used for various purposes.

The difference between the elongation percentage after breaking of the non-woven fabric and that of the elastic sheet corresponds to the difference between the first stress-lowering point of the composite elastic material and the second stress-lowering point thereof. Preferably, the difference is 50% or more.

The most simple morphology of the composite elastic material of the present invention is a two-layer structure in which a single non-woven fabric is bonded to a single elastic sheet, while the composite elastic material may be a sandwich structure of three layers, in which one elastic sheet is interposed between two layers of non-woven fabrics, each of the layer comprising one or more non-woven fabric, while it may be a four-layer structure in which two elastic sheets, to a side of each of which a non-woven fabric is bonded, are laminated and jointed to each other so that the other sides of the two elastic sheets, to which no non-woven fabrics are bonded, can face each other. In any case, upon at least one side of the composite elastic material, a non-woven fabric is naked.

Furthermore, when a non-woven fabric which is manufactured according to a water entanglement and has a two-stage elongation property as different in a stress level is used, a composite elastic material having an excellent stopper effect can be provided, in which the level of a strength against breaking on a first stress lowering which is caused by the breaking of the non-woven fabric is elevated.

According to the present invention, by properly selecting the following conditions, it is possible to enlarge the range of properties which an elastic sheet can have, and to further improve the functions thereof as an elastic material:
(1) To utilize the elongation property of a non-woven fabric having an elongation property so as to provide a composite material with such an elastic property as not to show the elongation property in a common state, and as not to activate the elastic property until the composite material is elongated.
(2) To use a non-woven fabric such as the one obtained by a water entanglement as the non-woven fabric having an elongation property, that is, to use a non-woven fabric having both an excellent elongation property and such a two-stage elongation that the stress is elevated again from a certain point of time.
(3) To adopt such a joint structure as a bond system that the bond sites are distributed in an approximately perpendicular direction to the elongating direction so as not to cause a resistance to the elongation.
(4) With respect to the bond sites to bond a non-woven fabric to the right side and the seamy side of an elastic sheet, to position the bond sites so that the bond sites on the right do not overlap the ones on the seamy side so as to prevent the occurrence of defective sites.

Through the consideration on the structure as mentioned above, it becomes possible to obtain a composite elastic material which has a low elongation property and a low residual strain, and is excellent in a stretch recovery property.

More particularly, Figure 2 shows an S-S curve of a composite elastic material which comprises a non-woven fabric and an elastic sheet and is similar to the above-mentioned one, when the composite elastic material was continuously elongated at a constant velocity until it has been fractured, in which the stress continues to increase until the elongation percentage approaches nearly 250%, while the breaking of the non-woven fabric is caused so as to rapidly lower the stress, when it has exceeded approximately 250%. However, the lowering of stress is stopped at a certain value, which state is maintained until the complete breaking is caused.

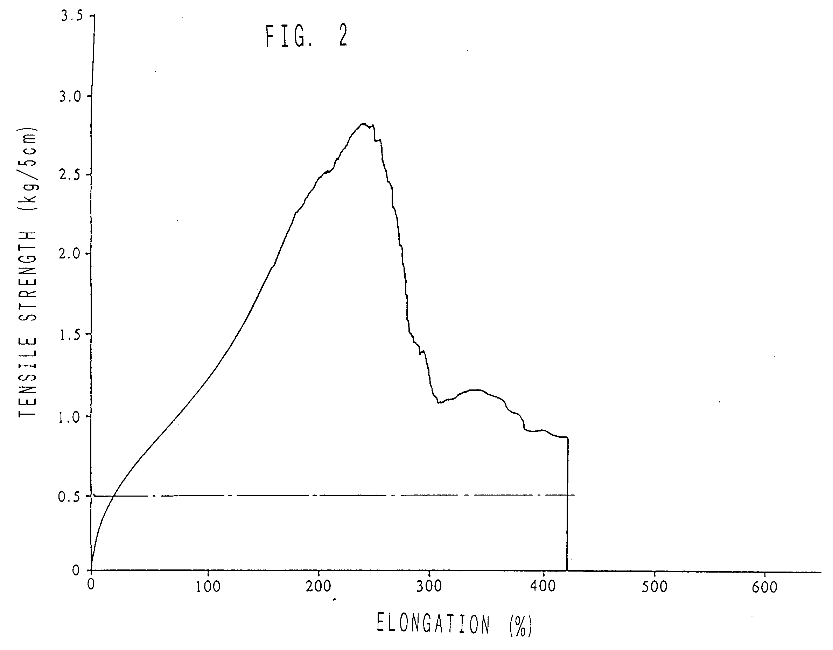

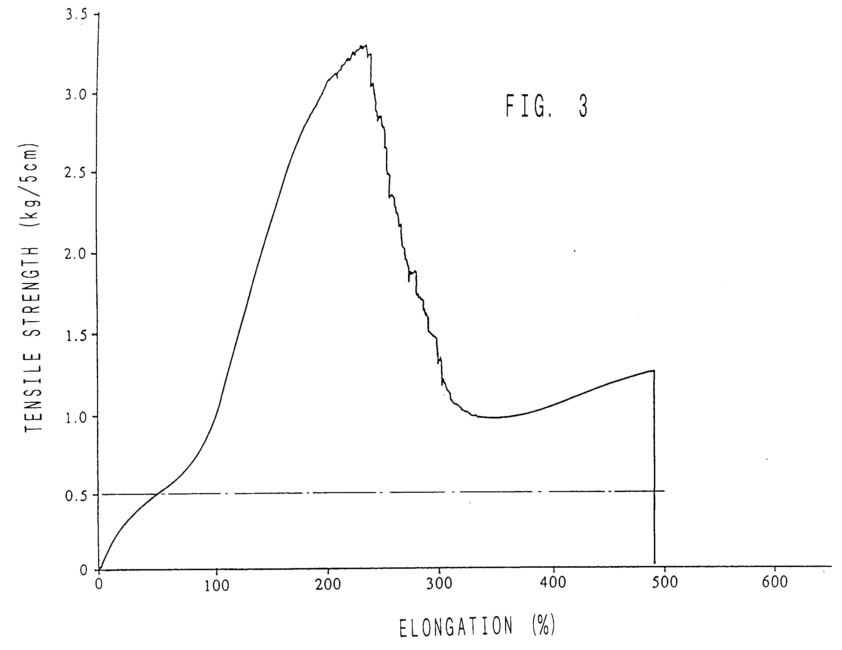

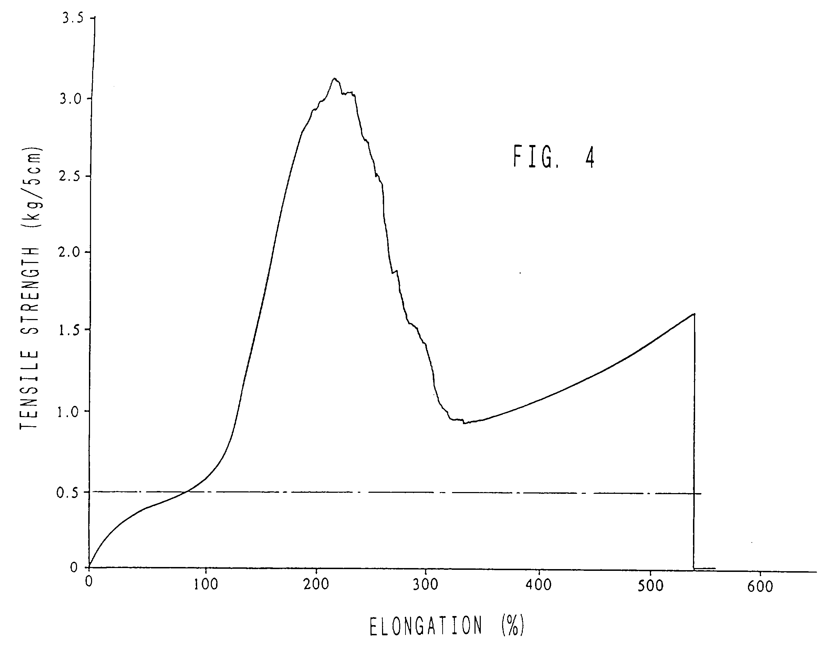

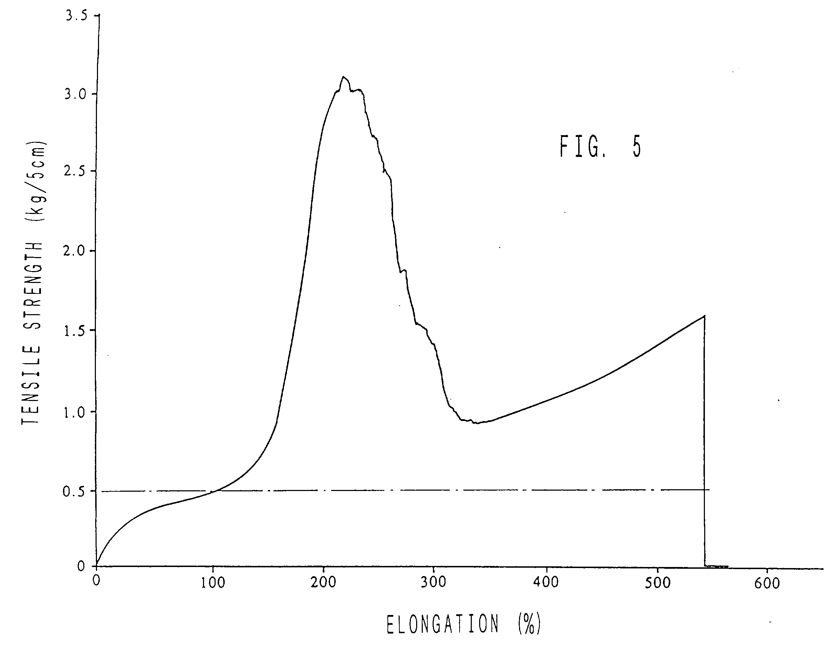

On the other hand, the composite elastic material as shown in Figure 2 is previously drawn under a condition of the elongation percentage after breaking of the non-woven fabric or less so as to activate the elongation, it provides a structure, thereby, a structure is provided by which the initial elongation and elastic modulus are lowered and the composite elastic material can is very easily elongated according to the predrawing percentage as shown in Figures 3, 4 and 5. For example, through such a predrawing, when the elongation percentage is 30%, the stress is lowered to 500g/5cm or less.

Figures 3, 4 and 5 show S-S curves of composite elastic material which were subjected to predrawing with percentages of 75% (which corresponds to about 30% of the elongation percentage after breaking of a non-woven fabric), 100% (which corresponds to about 40% of the elongation percentage after breaking thereof), and 150% (which corresponds to about 60% of the elongation percentage after breaking thereof), respectively. This predrawing is carried out preferably in the range of 30% to 80% of the elongation limit of a non-woven fabric. If the predrawing percentage is 30% or less, an adequate effect of the predrawing is not expected, while if the predrawing percentage is 80% or more, a care should be taken since breaking may occur in advance.

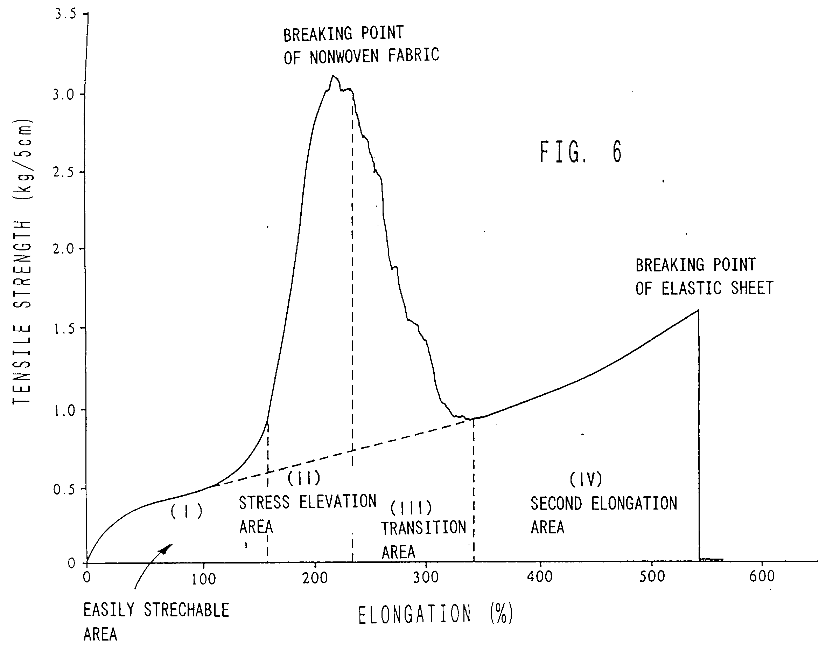

A composite elastic material is predrawn as mentioned above so as to become to show a characteristic S-S curve as drawn in Figure 6, which has such an area (I) having an easily stretchable property that the stress at the point of an elongation percentage of 30% is 500g/0.5cm or less; an area (II) in which the stress is rapidly elevated, which functions as an elongation resistance against the elongation limit of a non-woven fabric when it was further elongated; an area (III) which transits from a first stress-lowering point to a second stretchable area; and the second stretchable area (IV) which reaches a second stress-lowering point through the transition area.

When a composite elastic material having an S-S curve as mentioned above is applied to, for example, a waist band of an absorbent product, it can come to have such a stretching function as to be able to easily follow a motion on putting on; a stopper function by which the warning of breaking can be given on the basis of the starting of stress; and such a function as to provide room to prevent the falling also after breaking, together.

The change of S-S properties between before and after predrawing elucidates that a systematic change is made in the non-woven fabric itself, and/or at bond sites of the non-woven fabric and the elastic sheet. This change is useful for providing an excellent performance as an elastic material when the composite elastic material is used for various purposes.

Whether predrawn or not, the composite elastic material of the present invention shows an excellent elastic property as an elastic material in the elongation range of less than the first stress-lowering point.

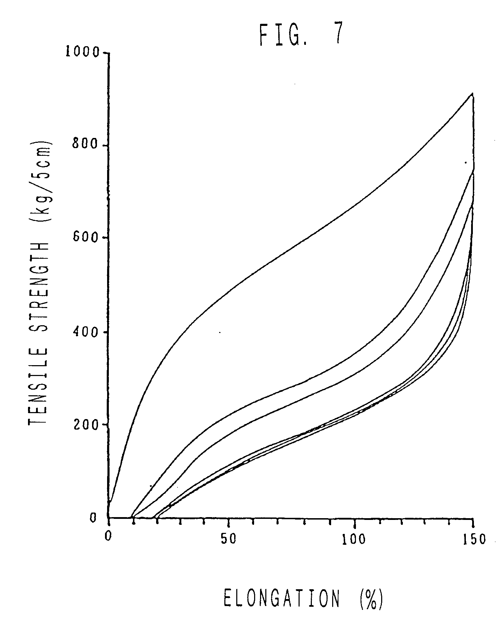

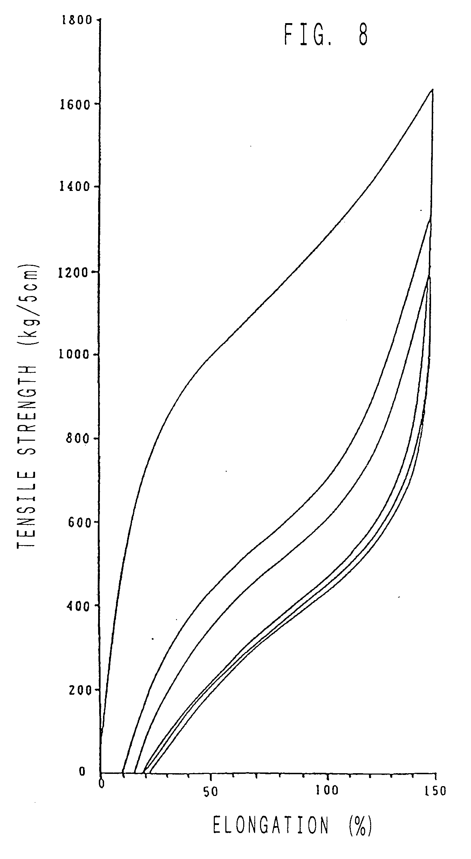

Each of Figures 7 and 8 shows an S-S curve of each of typical two composite elastic bodies of the present invention, when it was elongated and eased from a non-elongation state to 150% which corresponds to the elongation percentage after breaking of a non-woven fabric or less. These composite elastic bodies show a relatively high stress when first elongated, since the structural change of the elongation of two materials comprising the non-woven fabric and the elastic sheet is simultaneously caused. However, when the composite elastic bodies are second elongated, the resistances are sharply lowered and the elastic properties as an elastic sheet come to be revealed as these are, because the non-woven fabrics have been already elongated, to which changes on and after the third times also are similar. The revelation of these phenomena is referred to as "elongation activation" in the present specification.

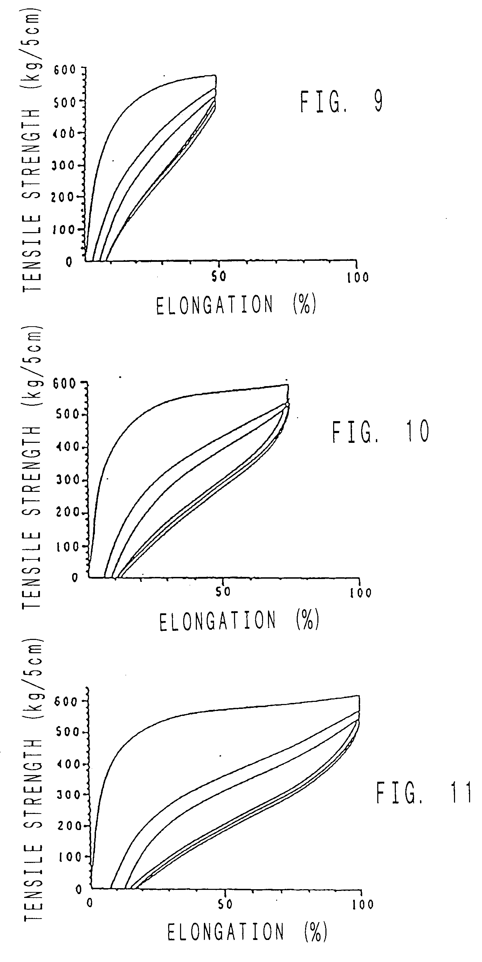

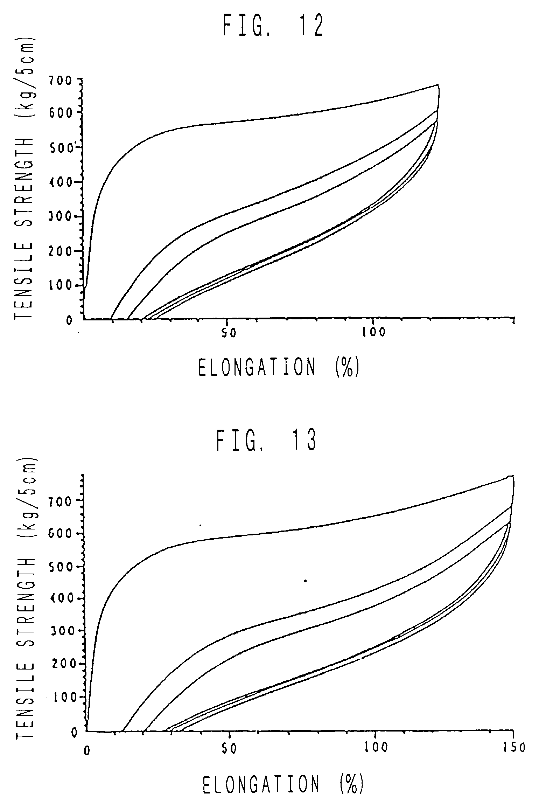

Another large characteristic of the elongation activation is that the elastic property is shown in the range of elongation percentage according to the elongation percentage, as shown in Figures 9 to 13. Therefore, when the material was put on the body, the material comes to be stretchable within such a range as to correspond to the motion of the body, and a flexible fitting-structure which has no uselessness can be provided.

In a case, the elongation activation is naturally carried out on putting on or using after the composite elastic material is provided for a product as mentioned above, while industrially an elongation activation is preferably carried out by positively incorporating a process for the elongation activation through predrawing, on or before manufacturing the product. For example, when a product in which the composite elastic material is used as a side elastic material or a waist gather of an absorbent product is manufactured, it is supposed there are following two cases: (1) when an elongation activation is previously carried out and an elastic material is used, and (2) when a composite elastic material is subjected to the elongation activation in a manufacturing process. In the former case, raw materials to be used come bulky so as to render the storing and conveying difficult, while in the latter case, there are no such problems. Therefore, it may be the that the latter is more preferable. A concrete manner comprises the steps of: widening and elongating a composite elastic material to provide it by using a simple belt tenter or a pin stenter apparatus when it is provided into a processing line; or incorporating a somewhat deep corrugated roll or gear roll in the processing line so as to provide a partial elongation treatment for the through the roll, and thereby the elongation activation is simply carried out. In this case, it is necessary to pay attention so that the surface of the elastic material is not injured by the non-woven fabric. With respect to a composite elastic material which was previously subjected to such elongation activation, S-S characteristics as shown in Figures 7 to 13 for a first elongation activation are vanished, and an S-S characteristic having an easily elongating property is shown from the start.

Desirable conditions as such composite elastic material of an elongation activation type are that a large stress is not impressed for an initial elongation as mentioned above, and resistance is rapidly elevated after the elongation percentage has passed a certain range, and thereby a resistance to an elongation before the composite elastic material has been fractured is rapidly elevated. Moreover, it is also important that even after a plurality of stretching were repeated, the similar elastic properties are maintained. After all, also that a residual strain is small is an important and fundamental performance.

The numeric representation of these desirable conditions are as follows.

When the composite elastic material was measured at intervals of 5cm width, an S-S curve on a first stretching has the following characteristics.

The measurement of physical properties as shown below was carried out on the basis of JIS (the Japanese Industrial Standards), which are commonly used in this field. The main points are as follows:
1. Test Samples
   Width : 5cm
   Length: 15cm
2. Conditions for Determining S-S curves
   Chuck Space : 10cm
   Loading speed: 20cm/min.
3. Cycle Tests
   The load-unload operation was repeated three times at an elongation percentage of 150% so as to obtain a hysteresis curve thereof. Stresses at 30% and 100% points from the final return point of the hysteresis curve were read. Incidentally, the following 5-minute intervals were provided as an easement time between each cycle:
   First Measuring → 5-minute Interval → Second Measuring → 5-minute Interval → Third Measuring.

(1) Stress at Elongation Percentage of 30%
   This shows an initial elongation stress, which is a force needed for an initial elongation on using, which needs to be controlled to a proper stress, because a too large stress provides a weighty touch and such a feeling as to be difficulty to elongate. From experience, it is desirably 1000g or less, preferably 800g or less, more preferably 600g or less.
(2) Stress at Elongation Percentage of 100%
   This stress is the one needed for an elongation activation, which is varied according to the working condition of an object that to what extent the object is elongated to be used. Since commonly a composite elastic material of the present invention is largely characterized when it is used at a high elongation, it was presumed that the composite elastic material is usually used at an elongation percentage of 100% or more, and therefore, the stress at an elongation percentage of 100% is selected as a point to be estimated.
   For the elongation activation of a composite elastic material of the present invention, a stress of 400g or more is needed at an elongation percentage of 100%, and therefore, when a stress of preferably 400g or more, more preferably 800g or more is selected, the elongation activation will be carried out by once elongating.
(3) Strength against Breaking
   The strength against breaking is 400g or more, it is usually satisfied. However, if it is desirably 600g or more, a safety from an unexpected breaking is elevated, because a resisting feeling which shows the elongation limit can be more clearly felt.
   When an elongation activation for such composite elastic material was carried out, a measured value of stress thereof is sharply lowered after the elongation activation. For example, when an elongation activation was carried out after a 150% elongation, such a stress as to elongate the composite elastic material by 150% or less twice or more is sharply lowered. In view of the purposes for use of a composite elastic material of the present invention, such lowering of the elongation stress is desirable.

Then, when a second S-S curve is determined by using a composite elastic material after an elongation activation was carried out by 150%, the following conditions are obtained.
(1) Stress at Elongation Percentage of 30%
   This stress needs to come to 500g or less through an elongation activation, desirably 400g or less, more desirably 300g or less. By selecting such conditions, even when the composite elastic material is applied to, for example, a baby product, it is possible to avoid suffering an excessive compression.
(2) Stress at Elongation Percentage of 30%
   The stress at an elongation percentage of 30% also is sharply lowered after an elongation activation so that it comes easy to elongate the composite elastic material, while such stress causes slipping down.

Therefore, 100g or more, more preferably 200g or more is demanded as the strength.

Furthermore, an important point as an aim of an elongation recovery power is that the recovery percentage is high. Namely, a structure having a lower residual strain is preferable. This elongation recovery percentage is commonly measured after the elongation recovery at a 150% point is repeated three times, so as to estimate the performance, and the recovery percentage represented under such conditions is 60% or more, preferably 70% or more.

Next, material components which a composite elastic material having such elongation recovery property comprises will be explained.

First, an elastic sheet to be used is selected preferably from raw materials which have an elongation property of 200% or more and an elongation recovery property of 60% or more. As a raw material having such performance, for example, a foam type such as urethane or a rubber latex; a styrene elastomer film such as an isoprene or butadiene synthetic rubber film, SIS, S.E.B.S., or S.E.P.S.; a polyolefin elastomer film; and a melt-blown elastomer non-woven fabric such as polyurethane, SIS, S.E.B.S. can be cited. In particular, it is preferabe to use a film, an article in the form of a net or a melt-blown non-woven fabric comprising a styrene elastomer such as SIS or S.E.B.S. having a good thermal bondong property, and a blended elastomer thereof.

A non-woven fabric, another components for the composite elastic material of the present invention, will be explained.

The non-woven fabric as used in the present invention has to have a large elongation property in a machine direction (MD) or a cross-machine direction (CD). In case of a non-woven fabric, it is inevitable that the non-woven fabric having an elongation property in both MD and CD somewhat sacrifices an elongation property in the CD, which is different from that of the non-woven fabric which is easily elongated in particular in the CD. A non-woven fabric which is commercially cheaply available will have a remarkably large elongation property in the CD. Examlples of such a non-woven fabric may inculde a non-woven fabric which is obtained by subjecting a parallel web to a water entanglement; a non-woven fabric which is obtained by opening and widening continuous tow fibers; a non-woven fabric which is obtained by subjecting a spun-bond or melt-blown to a treatment for orientating the fibers in parallel. When it is considered whether the non-woven fabric is commercially readily available, it can be roughly classified into the following three groups:

### Group 1

A non-woven fabric produced from a card web of which fibers are oriented in the MD and subjected to water entanglement, having the ratio of an elongation in the MD to the one in the CD (MD/CD ratio) of 2 or more, preferably 3 or more, and those obtained by drawing such non-woven fabric. This group will be explained below in more detail.

### Group 2

A non-woven fabric which was obtained by thermodrawing and parallelizing a spun-bond non-woven fabric, a melt-blown non-woven fabric, or a dry non-woven fabric in which the MD/CD ratio is 2 or more, preferably 3 or more, and fibers are oriented in the MD. In addition, a non-woven fabric which was obtained by heating a spun-bond comprising a composite fiber, and drawing it to the melting point of an easily meltable polymer; the composite fiber comprising a sheath part from an easily meltable polymer such as polyethylene or a PET derivative, and a core part from a thermally stable polymer such as polypropylene or PET. This non-woven fabric is in particular suitable for the present invention, because it is thin, improved in an orientation in a parallel direction, and has no feather-standing.

### Group 3

A non-woven fabric in which a spun-bonded non-woven fabric, a melt-blown non-woven fabric, or a dry non-woven fabric in which the ratio of MD/CD is 2 or more, preferably 3 or more, and fibers thereof are oriented in the MD was provided with many fine slits in the MD, i.e., in an perpendicular direction to the CD.

A concrete value of such stretchable property in the MD or CD is desirably 100% or more, more desirably 200% or more.

Due to such elongation properties of the non-woven fabric, it comes to be possible that the behavior of the elastic sheet is followed by the non-woven fabric.

Moreover, the non-woven fabric has another important performance besides such stretchable properties. It is that when an elongation thereof has exceeded a certain value, the non-woven fabric shows resistance to further elongation.

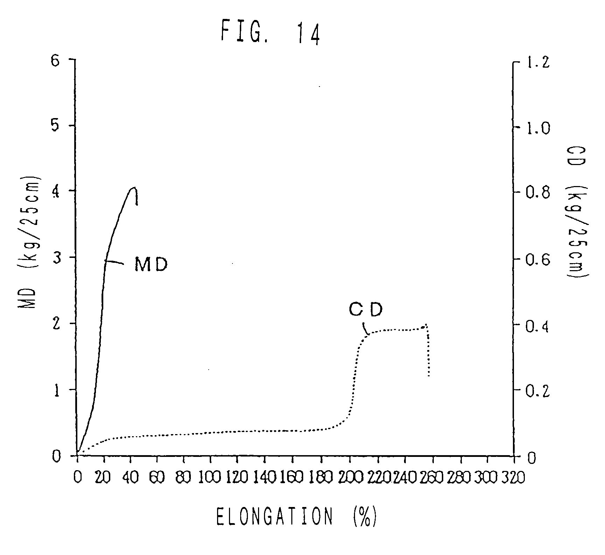

One of non-woven fabrics which can be in particular advantageously used for the present invention is a water entanglement non-woven fabric. As shown in Figure 14, the water entangled non-woven fabric shows a little elastic property in the MD, while shows a large elastic property in the CD, and when it was elongated to about 200% of the natural length thereof, the stress was once sharply elevated, and it was further elongated under the stress, and thereafter it has reached the breaking point (corresponding to an elongation percentage of about 260%). This elevation of stress at second stage will act as a brake before breaking. The non-woven fabric is designed so that such stress-elevation point is desirably higher than a 150% elongation, more desirably at a 150% or more.

In order to show such an optimum elongation performance, it is important how to combine the constitution of web fibers with the conditions of a water entanglement. For example, a non-woven fabric having the following constitution can be cited as the one which can satisfy the above-mentioned condition:
(1) Constitution of the Web
   - combining relatively short fibers having a length of approximately 25mm to 45mm with relatively long fibers having a length of 45mm to 60mm so as to form a staple fiber of a raw material; and
   - combining such fibers as to cause a wound-shrink (Kanshuku) by shrinking therewith.
(2) Selection of Stream Entangling Conditions
   After jointed all over the surface by using fine nozzles, the non-woven fabric was strongly jointed at the selected areas. For example, three-stage nozzles are used.
   - 1st Step: Nozzle Diameter: 0.15mm⌀
   Nozzle Spacing : 0.5mm
   Water Pressure : 30kg/cm²
   - 2nd Step: Nozzle Diameter: 0.15mm⌀
   Nozzle Spacing : 0.5mm
   Water Pressure : 50kg/cm²
   - 3rd Step: Nozzle Diameter: 0.25mm⌀
   Nozzle Spacing : 1.0mm
   Water Pressure : 60kg/cm²

Then, a water entangled non-woven fabric having a striped pattern which is elongated in the MD can be provided.

Another example of a non-woven fabric which can be advantageously used for the present invention is a composite non-woven fabric of the above-mentioned Group 2. This non-woven fabric is prepared by drawing a composite non-woven fabric which comprises a fibrous article comprising a jointing component (A) and a skeleton component (B), while heating at a temperature from the softening temperature or more of the jointing component (A) to within the stabilizing temperature area of the skeleton component (B); the component (A) having an easily thermoplastic property, the component (B) being relatively thermally stable as compared with the component (A). An easily stretchable non-woven fabric obtained according to such process has a stretchable property in one direction, and is excellent in a feeling aspect such as the touch. Therefore, a composite elastic material which is prepared by combining the non-woven fabric is quite suitable for an elastic material of a product such as a underwear or a sanitary article.

In order to provide a structure maintaining the morphology and nature of a non-woven fabric, and having an easily extensible property in the present invention, it is necessary to maintain a bond in machine and cross directions, at the same time to reorient constitutive fibers. In order to satisfy such conditions, the present invention adopts the means for heating a composite non-woven fabric which comprises a jointing component (A) and a skeleton component (B) as mentioned above so as to plasticize the jointing component (A), and for utilizing the fluidity thereof to shift a space between constitutive fibers by a drawing operation so as to reorient the fibers.

In the composite non-woven fabric which is used as a starting material, examples for combining the jointing component (A) having an easily thermoplastic property with the skeleton component (B) which is relatively thermally stable as compared with the jointing component (A) will be explained. Both the jointing component (A) and the skeleton component (B) are in the morphology of a filamentous, staple or fibril form fiber, examples of the types for combining the jointing component (A) with the skeleton component (B) are as shown in the following table:

| Types | (1) | (2) | (3) | (4) | (5) | (6) | (7) | (8) |
|---|---|---|---|---|---|---|---|---|
| Component (A) | PE | PE | EVA | PE | SEBS | PE/PET | SEBS | Nylon |
| Component (B) | PP | PET | PP | Nylon | PP | Poly-AN | Acetate | PET |

| | | | | | | | | |
|---|---|---|---|---|---|---|---|---|
| *Poly-AN means polyacrylonytrile | | | | | | | | |

Incidentally, in the above-mentioned combinations, at least the skeleton component (B) is a component which is preferably in the morphology of a staple fiber in which fibers are ready to be oriented by drawing, more preferably in the morphology of a continuous filamentous fiber.

As example of a combination, there are a single-layer composite non-woven fabric having two components of a component (A) and a component (B); and a non-woven fabric web comprising a jointing component (A) and a non-woven fabric web comprising a skeleton component (B) are layered into double-layer.

An examples of the single-layer non-woven fabric is a filament non-woven fabric comprising conjugated fibers of PET (polyester), such as a spun-bond non-woven fabric (ELVES) made by UNITIKA, LTD. is cited.

As exemplary examples of the double-layer non-woven fabric, the one in which melt-blown webs having an easily thermoplastic property are laminated to both sides of a spun-bond of PET so that the spun-bond is sandwiched between the melt-blown webs; and the one in which an acetate tow is opened, and PP (polypropylene) fibril fibers are laminated to both sides of the acetate tow as a nucleus so that the acetate tow is sandwiched between the fibril fibers are cited. Commonly, it is preferable to distribute the skeleton component (B) as a core, and the jointing component (A) as a surface area.

For the reasons above, it comes to be possible to maintain such a structure as to cause no lowering or no deterioration of fundamental physical properties, even if the surface of the non-woven fabric comes into such a state as to easily cause softening or fluidizing.

In order to form a structure which is excellent in a stretchable property while maintaining an adequate strength for users as aimed by the present invention, it is necessary that constitutive fibers are entangled in each other. Namely, the oriented state of a web which is suitable for the present invention is that of what is called spun-bond in which a web comprises filaments which are piled up in a loop state; or that of a web having a high randomness, in which a bundle of tow filaments is opened, widened and laminated; or the like. These webs have the property that it is easy to be reoriented by a thermo-drawing.

From these points of view, a non-woven fabric which is formed by thermally bonding parallel dry webs of short fibers which are already in an oriented state is not preferred very much. On the other hand, a non-woven fabric in which webs are jointed to each other in machine/cross directions by using a cross-lapper is not also preferred very much, since the entanglement in machine/cross directions is too strong.

In order to heat and draw a web having a property as mentioned above to reorient it so as to prepare a non-woven fabric which is excellent in a stretchable property in a cross direction, it is important to properly select drawing conditions, i.e., temperature conditions which are applied to the web on drawing; and conditions such as the sort of a heat medium which is used for heating.

### [Temperature Condition]

It is desirable that between jointing component (A) and a skeleton component (B) which a composite non-woven fabric comprises, only the easily thermoplastic jointing component (A) is plasticized, and drawn at a temperature range in which the skeleton component (B) is stable. Commonly, the temperature is properly in the range of about 90O°C to about 160O°C. Foe example, the temperature of a composite non-woven fabric comprising a combination of PE/PET is desirably in the range of about 100O°C to about 120O°C.

### [Heating Medium]

A most suitable heating medium is a fluid containing water, concretely, steam and hot water. Drawing in such heating medium does not cause mutual joints between constitutive fibers of webs, while provides an easily stretchable non-woven fabric having a soft finish. The lowered denier of the constitutive fibers by drawing also contributes to the softening.

Steam or hot water is desirably used by combining hot water with hot air, or by combining steam with hot air rather than by itself. In particular, when drawing is carried out with a multistage, it is desirable to use a combination of plural heating media.

Incidentally, a usual heating roller or a dry hot air as a heating means is not preferred, because such means is in danger of mutually thermo-melting constitutive fibers of the webs.

When drawing is carried out under heating, if widening is carried out as a pretreatment, and thereafter drawing is carried out, it is possible to more easily obtain an easily stretchable non-woven fabric having an excellent quality. In this case, so large widened percentage is not necessary, and a widened percentage in the range of merely about 110% to about 150% suffices therefor. For such widening, expander rolls or grid gears can be used. It is one of recommendable and effective methods that such widening is carried out in hot air at a temperature of 70O°C to 80O°C, and then drawing is carried out in a saturated steam.

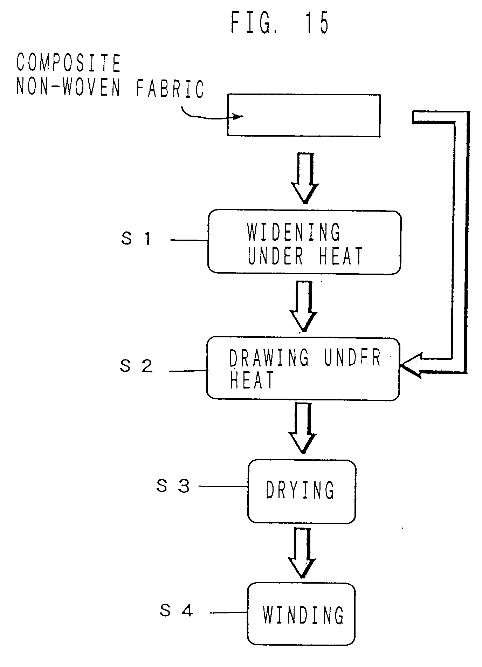

Figure 15 is a flow chart showing one example of a process of a method of the present invention. In Figure 15, an original cloth comprising a composite non-woven fabric is first widened under a steam heating (Step S1), and drawn in approximately perpendicular direction to a widening direction under a steam heating (Step S2), and dried (Step S1), and finally wound into a roll (Step S4). According to conditions such as the properties of the original cloth to be used, the widening step may be omitted so as to directly carry out the drawing step (Step S3) without widening.

When such drawing operation is carried out, the width is apt to shrink, which will cause a convergence wrinkle. When the convergence wrinkle is not preferred, the width can be controlled by using a tenter or the like on drawing it so as to prevent the occurrence of such convergence wrinkle. On the other hand, however, the occurrence of the convergence wrinkle in a machine direction can be controlled to intentionally provide a micro corrugated structure so as to form a non-woven fabric structure which is more easily stretchable in a cross direction.

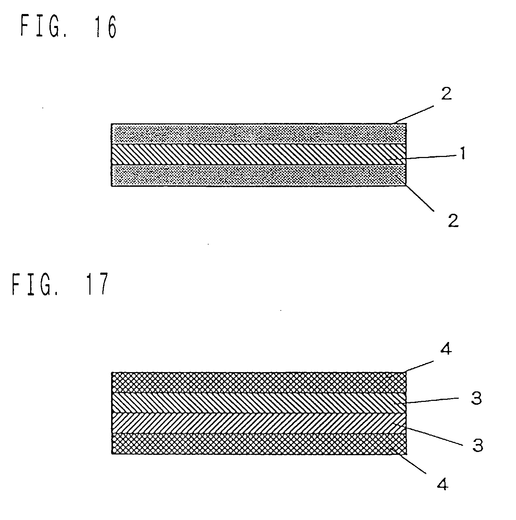

Figure 16 is a drawing of longitudinal section which shows an example of the constitution of a composite non-woven fabric suitable to use as an original cloth according to a method of the present invention. An easily stretchable non-woven fabric as shown in Figure 16 has the structure of a double-layer non-woven fabric in which a PE melt-blown web layers (2) of a jointing component (A) are laminated to both sides of a PET spun-bonded web layer (1) of a skeleton component (B) which is positioned in the center. As shown in Figure 17, an alternative structure may be selected, in which two sets, in each of which a PP burst fiber web layer (4) of a jointing component (A) is laminated to one side of a PET web layer (3) of a skeleton component (B), are laminated so that the layers (3) of a skeleton component (B) can face each other.

In order that a composite non-woven fabric to be used for a method of the present invention can endure a widening and/or a strong drawing which the composite non-woven fabric suffers in a process for manufacturing an easily stretchable non-woven fabric, so as to be desirably reoriented, it is desirable that the layers of the composite non-woven fabric are moderately bonded to each other.

Even if one non-woven fabric is bonded to another non-woven fabric, if the degree of bond is proper, that is, if when the fibers are oriented, these non-woven fabrics are bonded to each other so that they can have some degrees of freedom, such composite non-woven fabric also can be used. However, in order to increase the homogeneity of whole drawing, a composite non-woven fabric which is bonded with many small bond points is advantageous. In this case also, if the fusion at each bond point is too strong, since besides the bond between a jointing component(A) and another jointing component (A), and the bond between a jointing component (A) and a skeleton component (B), the mutual strong bond between skeleton components (B) is caused, the composite non-woven fabric may be weakened at the portion so as to lose the drawing property.

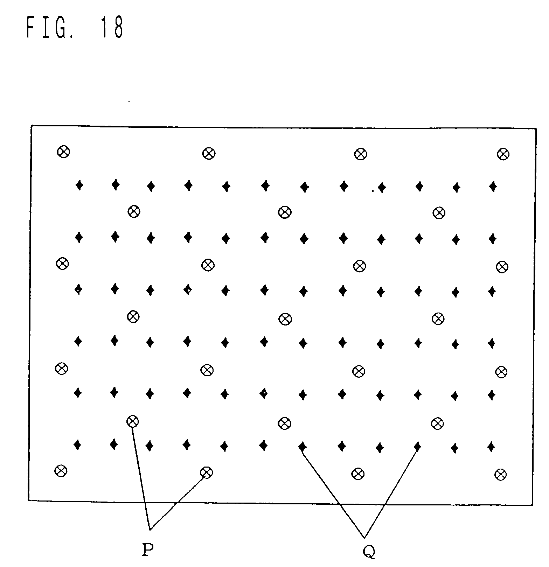

In order to avoid such inconvenience, for example, as shown in Figure 18, it is one of effective means to adopt the two-phase bond structure in which two types bond-points (P) and (Q) are distributed with a proper space; the (P) and (Q) being different from each other in degree of bonding. In the bond-point (Q), the temperature and pressure on fusing are controlled so as to bond merely jointing components (A) to each other, and a jointing component (A) to a skeleton component (B), while in the bond-point (P), bonding is carried out under such a temperature and pressure condition that the skeleton components (B) also can be bonded to each other.

A composite elastic material of the present invention has a structure in which the above-mentioned non-woven fabric having an elongation property is bonded to either or both of the surfaces of an elastic sheet. In this bond system, the following factors come important, because an applied bond system makes a difference of performance between the resultant composite elastic bodies.
(1) To make the easily elongating direction of a non-woven fabric correspond to the one of an elastic sheet so that the non-woven fabric is bonded to the elastic sheet;
(2) To adopt such a bonding pattern that bond sites do not impede the elongation of composite elastic bodies as much as possible; then, it is desirable to make the number and area of bond points in an elongation direction few as much as possible; such bonding can be easily realized by distributing bond points so as to come to be preferably in the range of 90O ±10O to the elongation direction of a non-woven fabric; and
(3) That when a non-woven fabric is bonded to both surfaces of an elastic sheet, a large difference is caused in an elastic property according to the arrangement of bond sites which are formed between one non-woven fabric and one elastic sheet, and between the other non-woven fabric and the other elastic sheet.

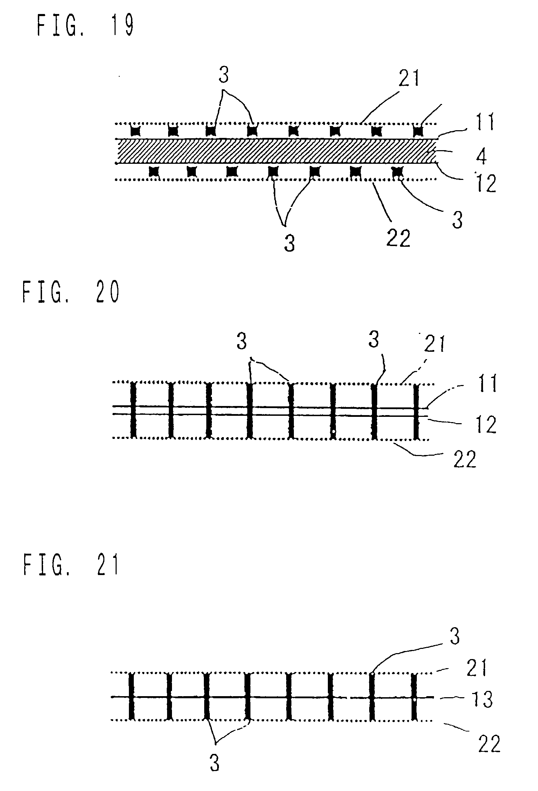

In a both-sides bond structure of an elastic sheet and non-woven fabrics, assuming that the thickness of the elastic film is 50µm, both one film sheet having a thickness of 50µm and two film sheets having a thickness of 25µm can be used. Then, as a combination of the elastic sheet and the non-woven fabrics, three cases can be conceived as shown in Figures 19 to 21:
(1) the case in which two elastic sheets (11, 12) each of which has a thickness of 25µm are bonded to non-woven fabrics (21, 22) with bond sites (3), respectively, so as to provide two composite sheets (20A, 20B), which are laminated to each other, and the elastic sheets (11) are bonded to each other by means of hot press (4) or the like (as shown in Figure 19);
(2) the case in which two elastic sheets (11, 12) each of which has a thickness of 25µm are bonded to non-woven fabrics, respectively, so as to provide two composite sheets, which are laminated to each other, and the elastic sheets are bonded to each other with bond sites (3) by means of hot press or the like (as shown in Figure 20); and
(3) the case in which two sheets of non-woven fabrics (21, 22) are laminated to both sides of one elastic film sheet (13) having a thickness of 50µm, and the non-woven fabrics (21, 22) and the elastic film sheet (13) are bonded to each other with bond sites (3) (as shown in Figure 21).

Then, an S-S curve of a combination having a structure as shown in each of Figures 19 to 21 was determined, from which it has been found that an elastic recovery property was elevated in the order of the cases (1), (2) and (3), when the length after easing the stress after elongating the combination by 100% was compared with the length before elongating the combination.

In particular, in case of Figure 19, the bond sites (3) of the first set comprising the elastic film (11) and the non-woven fabric (21) and the bond sites (3) of the second set comprising the elastic film (12) and the non-woven fabric (22) are arranged by shifting the phases thereof so that the position of the bond sites (3) of the first set and that of the second set are not overlapped each other. In this case, a tensile strength comes preferable because the position of the bond sites (3) on one side of the combination is different from the one on the other side thereof.

In a composite elastic material of the present invention, the morphology of bond sites for a non-woven fabric and an elastic material also one of important factors. In order to obtain an optimum S-S characteristics, such a bond morphology that neither the characteristics of the non-woven fabric or those of the elastic sheet is reduced. The condition necessary for such bonding is that the bond sites are discontinuous in a desired elongating direction. Namely, a non-bond site is positioned between bond sites which are adjacent to each other so that the non-bond site provides an adequate elongation property for the composite sheet. Furthermore, with respect to an perpendicular direction to the elongatable direction, each bond sites are preferably discontinuous not to check the whole flexibility.

A exemplary pattern of the bond sites which satisfies such conditions is a set of dots in any form such as a circle, a square and a polygonal shape. Furthermore, these dots may be homogeneously distributed all over the surface of the composite elastic material, while it is also effective to distribute a set of the bond sites having an optional number according to an optional pattern.

From an industrial point of view, it is advantageous to elevate manufacture efficiency that two relative thin sheets, in each of which a non-woven fabric is laminated to mere one side of an elastic sheet, are prepared, and then laminated and jointed to each other so that the elastic sheets can face each other, so as to prepare a composite elastic material to both sides of which the non-woven fabrics are laminated. In particular, when a polystyrene elastomer film such as SIS or S.E.B.S. was used as an elastic sheet, since the elastomer film is rich in adhesive property, it is possible to manufacture a stable both-sides jointed body merely by laminate the two films to each other. Thereby, the productivity is remarkably improved, and the cost can be remarkably lowered.

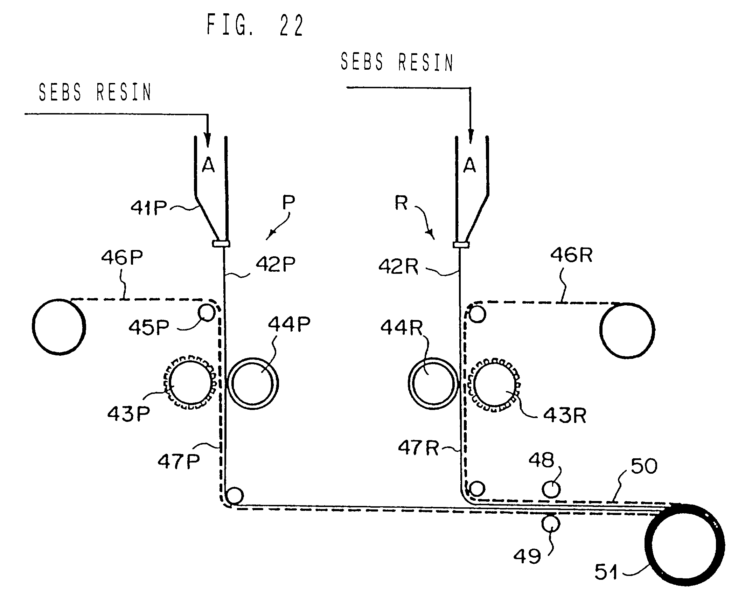

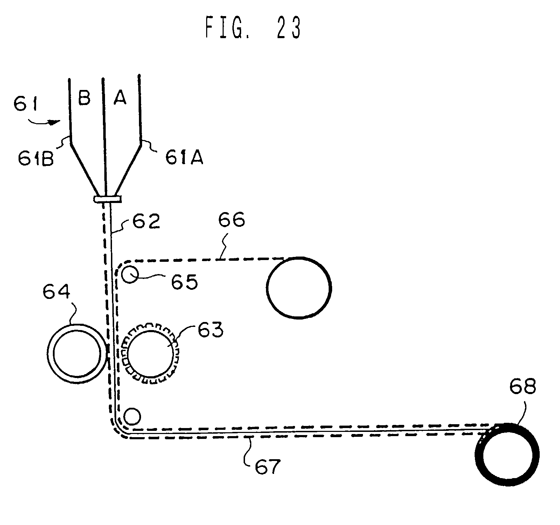

On the other hand, however, an self-adhesive property of this type of elastic film advantageously functions for jointing to a non-woven fabric, while the self-adhesive property is apt to cause adhesion when the surface of the film touched the surface of a roller or the like in a working process therefor, which causes a trouble on a process. Such trouble can be avoided by adopting a process as shown in Figure 22 or 23.

In a process as shown in Figure 22, two set of sections (P) and (R) for manufacturing composite sheets are used, the set being positioned in tandem to each other.
The first section (P) is provided with an extruding machine (41P) which contains a material for forming an elastic sheet, such as an S.E.B.S. resin. A film (42P) which was extruded by the extruding machine (41P) is laminated to a spun-lace non-woven fabric (46P) which has been guided by a guide roller (45P) on the way to a nip which is positioned between a heated embossing roller (43P) and a chilling roller (44P), so as to form a first composite sheet (47P).

A second section (R) for manufacturing a composite sheet has substantially the same constitution as that of the above-mentioned first section (P) for manufacturing a composite sheet, and thereby a composite sheet (47R) is formed; the composite sheet (47R) having the same structure as that of the first composite sheet (47P), except that a non-woven fabric (46R) is laminated to a film (42R) so that the relative position of the non-woven fabric (46R) and the film (42R) is opposite to that of the second composite sheet (47P) and the film (42P). In the second section (R) for manufacturing a composite sheet, the same parts as in the first section (P) for manufacturing a composite sheet are shown by the same numerals provided with (R).

Two composite sheets (47P) and (47R) are laminated to each other into such a position that the films (42P) and (42R) thereof can face each other, and guided to a nip between a pair of press rollers (48, 49). Each of the press rollers has the surface of which is chromed and has an ordinary temperature, through which two elastic sheets which have been introduced to the nip are crimped. According to the crimping, films (42P) and (42R) which face each other are bonded to each other by their own adhesive property. As a result, a composite elastic material (50) having a four-structure is wound by a product roller (51); the four-structure comprising a set of two elastic films and two non-woven fabrics which are bonded to both sides of the set of two elastic films according to a desirable bond-pattern.

When a product in which a non-woven fabric is jointed to only one side of an elastic sheet is desired, it may be necessary to conceive an alternative design so as to lower the self-adhesion of this side, since it is inevitable that the elastic sheet touches a roller or the like.

Figure 23 shows a process in which the above-mentioned points are taken into account. In Figure 23, a film coextruding machine (61) has two chambers (61A, 61B) which are discrete from each other, one chamber (61A) contains a material to form an elastic sheet, such as an S.E.B.S. resin (A), while the other chamber (61B) contains an S.E.B.S./E.V.A. blended resin (B). These two type resins are extruded as a film from nozzles which are provided for each chamber, respectively, and are touched each other when the resins are not yet solidified just after extruding, so as to form a combined coextruded film (62) having a two-layer structure.

This coextruded film (62) having the two-layer structure is introduced approximately straightly downward, and introduced to a nip between a heated embossing roller (63) and a chilling roller (64), and in process thereof, a spun lace non-woven fabric (66) which has been guided by a guide roller (65) is laminated to the side of the single layer of the S.E.B.S. resin of the film (62).

Since a heated embossing roller (63) has an embossing surface corresponding to a desirable bond pattern, the non-woven fabric (65) is bonded to the coextruded film (62) which was introduced to the nip between the heated embossing roller (63) and the chilling roller (64) with a desired pattern so as to form a composite elastic sheet (67). The resultant composite elastic sheet(67) like this is wound by a product roller (68).

As a E.V.A. as used for an S.E.B.S./E.V.A. blended resin (B), the one containing a large amount of vinyl acetate is advantageous in order to provide an excellent elastic property, and in view of an elastic recovery property, it is desirable that the thickness of a blending layer is thin as much as possible.

With regard to a bond pattern for a non-woven fabric and an elastic sheet, in a composite elastic material in which a non-woven fabric is laminated to only one side of an elastic material, or the non-woven fabric is laminated to both sides thereof as shown in Figures 20 to 22, bond sites (3) are shown as a band which is extended in an approximately perpendicular direction to the easily elongating direction of the elastic sheet and the non-woven fabric, preferably in a direction of 90⁰±10⁰. Such band-like bond sites may be the ones which can bond the elastic sheet to the non-woven fabric within a determined area with no space, or may be a line of bond sites in which many bond sites are oriented in a determined direction; the bond sites being in the morphology of lines or dots which are distributed within a determined bond area and have an optional pattern.

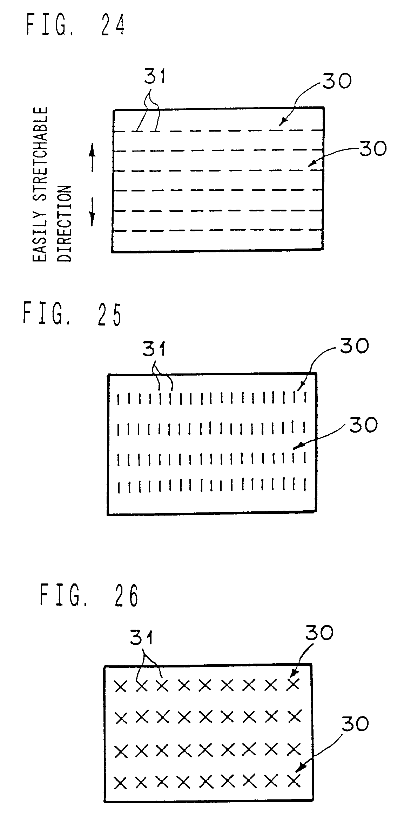

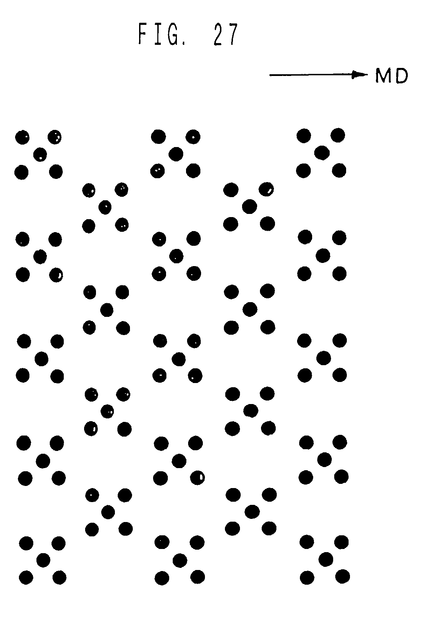

Each of Figures 24 to 28 shows an exemplification of the pattern of the line of bond sites comprising a gathering of discontinuous bond sites. Namely, in Figure 24, the plurality of lines (30) of bond sites are formed; the lines (30) being positioned in an approximately perpendicular direction to an easily elongating direction of a composite elastic material at a proper space, and extending in a parallel direction to each other. In an example as shown in Figure 25, many bond sites (31) which a line of bond sites comprises are oriented in an approximately perpendicular direction to the machine direction of the line of bond sites. Furthermore, in case of Figure 27, each bond sites (31) has a shape which is similar to the mark "X". Each bond sites (31) may have any shapes besides the shown one.

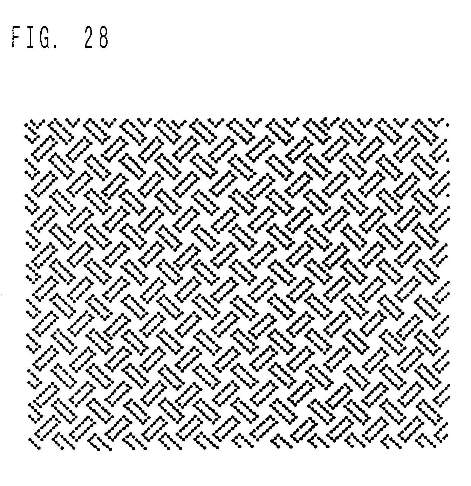

Alternatively, a pattern in which fine dots are arranged with a proper density along the points of a lattice which comprises two sets of parallel lines which cross each other at a proper angle to each other, as shown in Figure 27; and a pattern in which rectangles are arranged so as to be alternately oriented at right angles to each other, and fine dots are arranged along the four sides of each of the rectangles, as shown in Figure 28 also are preferable.

At bond sites, by which an elastic sheet and a non-woven fabric are bonded together, the elongation properties of these materials are made to be remarkably reduced so as to substantially have non-elongating properties. Therefore, when the lines (30) of bond sites were arranged so as to extend in an approximately perpendicular direction to the easily elongating direction thereof as shown in Figure 24, the elongating property in the easily elongating direction is little changed, but when arranged so as to extend in a parallel direction thereto, the elongating property is sharply lowered, even if the materials have a large elongating property in the parallel direction also. Therefore, a composite elastic material as shown in Figure 25 is little elongated in the longitudinal direction of the lines (30) of bond sites.

On the other hand, with respect to an perpendicular direction to the lines (30) of bond sites, in the precess in which the composite elastic material is elongated in the easily elongating direction, the composite elastic material is unrestrictedly elongated until it reaches the elongation limit of the non-woven fabric. Then, when the tensile force of the composite elastic material is eased at the elongation limit, both the elastic sheet and the non-woven fabric are recovered to the original length thereof. However, the composite elastic material is further elongated over the elongation limit of the non-woven fabric, although the elastic sheet having a remarkably high elastic limit is not changed, the non-woven fabric is elongated. As a result, the elastic recovery property of the composite elastic material is lost, and thereafter even if the tensile force is eased, the elastic sheet is recovered to the original length, while the non-woven fabric is left in an elongated state as it is. Thereby, when the composite elastic material has been recovered to the original length, the length of the non-woven fabric comes longer as compared to the one of the elastic sheet, the non-woven fabric comes in a baggy state between the lines of bond sites adjacent thereto.

Thus, after the non-woven fabric was elongated, when the composite elastic material is again elongated in the easily elongating direction, it comes possible to elongate the composite elastic material by a remarkably small force as compared to the tensile force which was necessary for first elongating the composite elastic material over the elongation limit of the non-woven fabric, which is an elongation activation as mentioned above.

As can be taken from the above-mentioned explanation, the elongation property of the composite elastic material in the direction in which the bonds sites or the lines of bonds sites are extended is remarkably small as compared to the one in the easily elongating direction thereof, which means that the elongating property of the composite elastic material also can be restricted only in a desirable direction by newly providing bond sites or lines of bond sites under a particular condition; the composite elastic material comprising the non-woven fabric and the elastic sheet having a large elongating property in any directions.

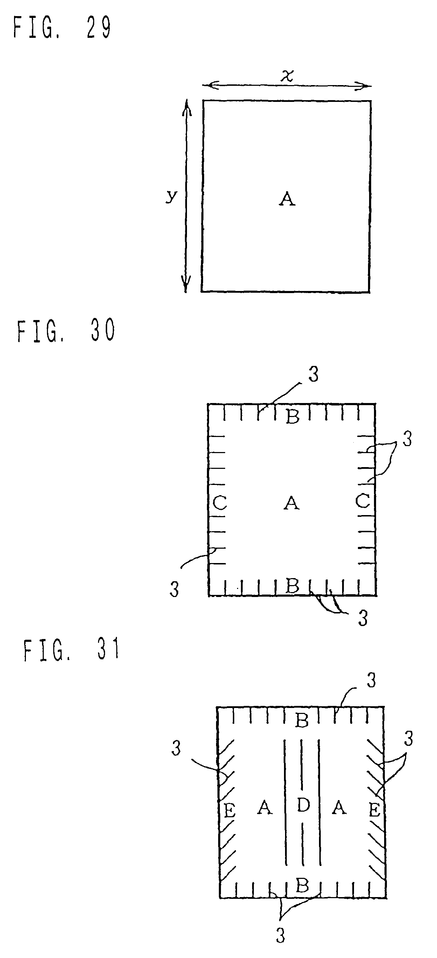

Figure 29 shows an approximately rectangular composite elastic material into which a non-woven fabric and an elastic sheet having largely elongating properties in both "x" and "y" directions were combined. With respect to such composite elastic material, in Figure 30, areas (B and C) extending along the four sides thereof are newly provided with linear bond sites (3) which extend in an approximately perpendicular direction to each of the sides. In an area (A) of the composite elastic material, in which no linear bond sites (3) are provided, the composite elastic material can be extended in any directions, while in the area (B) it has an elongating property only in an "x" direction, while in the area (C) it has an elongating property only in a "y" direction. Moreover, as shown in Figure 31, when in areas (B) which extend, respectively, along two sides which are situated at both sides in the "y" direction, and in an area (D) which extends in the "y" direction in the center part in the in an "x" direction, plural linear bond sites (3) which extend in each direction, respectively, are provided; and in areas (E) which extend, respectively, along two sides which are situated at both sides in the "y" direction, linear bond sites (3) which are inclined at an angle of about 45 degrees to the sides, respectively, are provided, the areas except the area (D) show a largely elongating property in the "x" direction, while the area (E) shows an elongating property only in an inclining direction.

When a composite elastic material in which the direction of an elongating property thereof was specified by adding linear bond sites thereto as shown in Figure 30 or 31 is used as a top sheet or back sheet of a diaper, which has an elastic property, "B" parts, "C" and "E" parts, and "A" parts may be made to correspond to an elastic material of the waist, an elastic material for surrounding the leg-hole, and the whole elasticity, respectively, so as to be contributed to a design of an article which is excellent in a morphology follow up property.

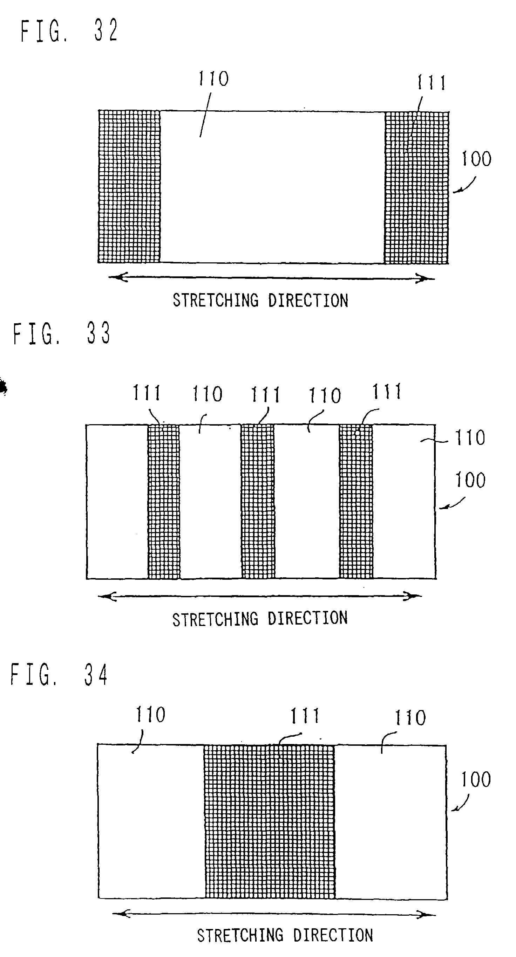

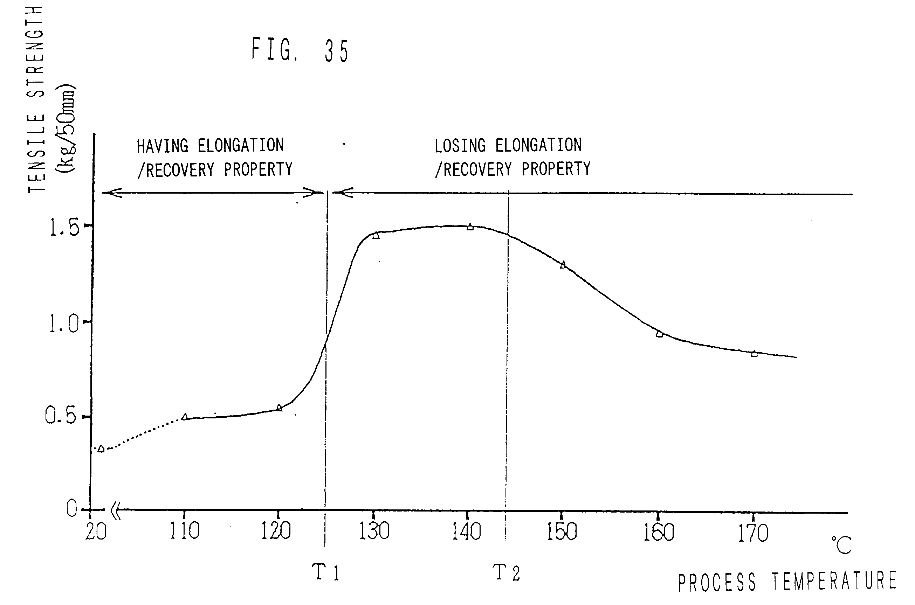

Figure 32 shows an composite elastic material (100) according to the basis of another mode of the present invention. This composite elastic material has an elongating property only in one direction, and when the composite elastic material was further and partially subjected to a thermo-compression bonding treatment, a first area (110) which is positioned in the center has a largely elongating property which the original composite elastic material has, since the first area (110) is not subjected to a new thermo-compression bonding treatment, while a second area (111) comes to hardly have an elongating property by a new thermo-compression bonding treatment. On the other hand, the bond strength of the second area (111) is strengthened by the treatment.
Furthermore, in Figure 33, three band-like areas (111) having a small elongating property are arranged with a prescribed space. Moreover, in Figure 34, areas (110) having large elongating properties are provided on both the sides of a band-like area (111) having a small elongating property. In any cases, a part having a small elongation is the one which was subjected to a new thermo-compression bonding treatment. Each of the elastic sheet and the non-woven fabric in this example is formed from a material which is easily melted by heating. A non-woven fabric corresponding to such purposes is formed, for example, by combining polyester and polyethylene, and comprises a conjugated resin web which comprises a sheath of polyethylene and a core of polyester; while an elastic sheet is a film comprising S.E.B.S.
(styrene-ethylene-butadiene-styrene block copolymer), with which the non-woven fabric is combined. A composite elastic material which was obtained like this is remarkably excellent in an ultrasonic sealing property and/or a heat sealing property, and shows an excellent performance on a high-speed manufacture.

Figure 35 is a graph which shows the relationship between a working temperature and a tensile strength, which were measured while a composite elastic material were subjected to a thermo-compression bonding; the composite elastic material being formed by laminating an elastic sheet comprising an SIS film to a non-woven fabric comprising a water entangled PET fiber and being partially subjected to a thermo-compression bonding. In this graph, the symbols "T1" and "T2" represent the melting start temperature of SIS which the elastic sheet comprises, the melting start temperature of PET which the non-woven fabric comprises, respectively. As can be taken from Figure 35, at a temperature of T1 or less, a thermo-compression bonding between the elastic sheet and the non-woven fabric are little carried out, and an elongation recovery property is observed, but a tensile strength is low. However, when a thermo-compression bonding was carried out within a temperature range between T1 and T2, at least a part of the elastic sheet was melted and bonded to the non-woven fabric, and thereby the elongation recovery property is lost, but the tensile strength is sharply elevated. Furthermore, when a thermo-compression bonding was carried out at a temperature of more than T2, both the elastic sheet and the non-woven fabric are melted, and the composite elastic material has little an elongating property in any directions.

Again, in Figures 32 to 34, a composite elastic material having such properties can be obtained by heat-treating a composite elastic material which has been formed at once as mentioned above, and it can be formed also by thermo-fusing and bonding an elastic sheet and a non-woven fabric which were directly laminated to each other so that the directions having elongation properties thereof can correspond to each other, under a different conditions between the area (110) and the area (111). Namely, a composite elastic material having a desirable elongating property can be formed by partially subjecting the elastic sheet and the non-woven fabric to a thermo-compression bonding at a temperature between T1 and T2 for the area (110) having a large elongation property, and by wholly subjecting them to a thermo-compression bonding at a temperature T1 or more for the area (111) having a small elongation property. This composite elastic material has the characteristics that since the elongation property of the area (110) having a large elongation property is restricted or controlled by the one of the small elongation property of the area (111), an elongation property in a prescribed elongating direction is large, while an elongation property in another direction is very small. Incidentally, if a thermo-compression bonding is wholly carried out at a temperature of T2 or more, such parts become fragile.

A composite elastic material in which an area having a large elongation property and an area having a small elongation property are mixed according to a desirable pattern can be applied for various purposes. As one example, a composite elastic material in which areas (111) having small elongation properties are formed on both the sides of an area (110) having a large elongation property as shown in Figure 32 can be applied to a tape-less type (pants type) of absorbent product.

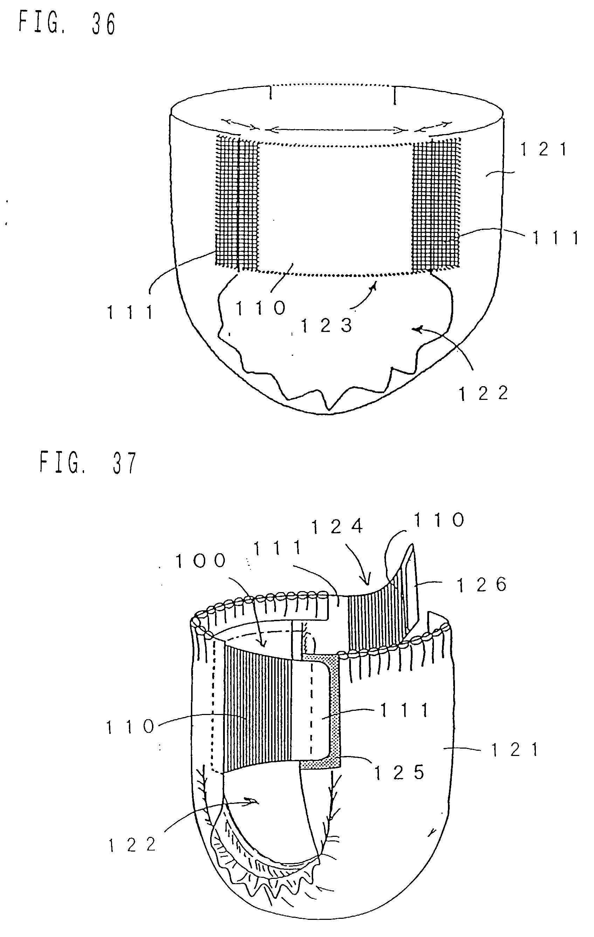

An absorbent product as shown in Figure 36, i.e., a tape-less type of diaper has a structure in which a main body (121) is bent into an approximate U-shape in the center thereof, and the opposite side-hems are connected to each other with a side panel (123) comprising a composite elastic material (100) as shown in Figure 33, except for parts forming leg holes (122); the main body (121) having a structure in which an absorbent is housed between a top sheet which is transparent to liquid and a back sheet which is opaque to liquid. This composite elastic material (100) is bonded to the main body (121) at an area (111) which is positioned on both the ends thereof and has a small elongation property, while is not bonded thereto at an area (110) in the center, which has a large elongation property. Therefore, the large elongation property of the area (110) in the composite elastic material (100) is not impeded, and the composite elastic material (100) can function as a side panel, and is strongly bonded to the main body (121) at the area (111) which has a large strength at both the ends thereof.

Moreover, Figure 37 shows a tape type of diaper having a structure in which a side band (124) comprising a composite elastic material (100) as shown in Figure 32 is provided for a main body (121). This side band (124) is bonded to the main body (121) at an area (111) having a small elongation property at one end of the composite elastic material (100), while a fastener (126) is provided at an area (111) having a small elongation property at the other end thereof; the fastener (126) being connected to a bond area (125) which is provided for the main body (121) so as to be removable. The diaper having such structure can be easily removed by unfastening the fastener (126), and furthermore, the waist portion of the diaper can be fitted to the waist of a wearing person by the large elongation property of an area (110) in the same way as the one in Figure 36.

The composite elastic material (100) may be predrawn at a stage before applying to the main body (121) of the diaper, or alternatively, may be predrawn after applying to the main body (121) by using a predrawing machine which was incorporated in the line for manufacturing a diaper.

The above-mentioned explanation has been done about examples for stretching mainly in the CD. However, it is also possible to apply to stretching in the MD, and moreover, it is also a significant technology to manufacture a composite elastic material having an elastic property in both CD and MD in combination with a publicly known S.B.L.

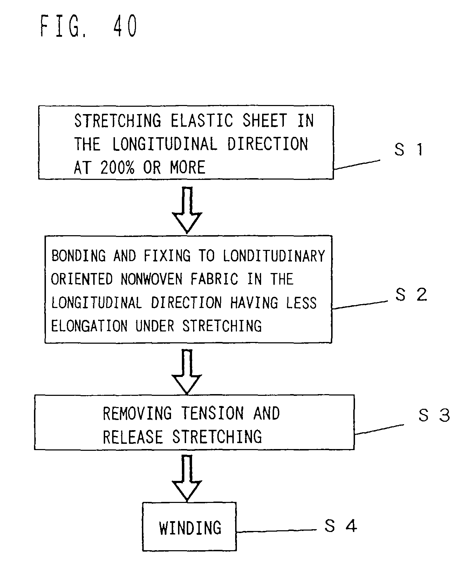

Figure 40 shows a process for manufacturing a composite elastic material having an elastic property in both CD and MD by combining an S.B.L. with a non-woven fabric having an easily stretchable property in the CD. In this process, an elastic sheet is elongated by 200% or more in a machine direction (Step S1), and a non-woven fabric having a small elongation property in a machine direction is laminated to the elastic sheet in such a state that the non-woven fabric is elongated by an elongating percentage within the elongation limit thereof in a machine direction, so that the elastic sheet and the non-woven fabric are jointed to each other by any means (Step S2), and then, the elongation of the jointed sheet is eased (Step S3), and the obtained composite elastic material is wound so as to be provided as a product (Step S4).

It is one of the advantageous effects of the present invention that a composite elastic material having an elastic property in both machine and cross directions can be easily provided according to such a method.

### Examples

Examples of the present invention will be explained as follows.

### (Example 1)

### 〈Manufacture of Nonwoven Fabric Having Elongation Properties〉

50 parts of a polyester fiber (1.5d × 35mm) and 50 parts of an easily thermoplastic conjugated fiber "Melty" (2d × 51mm) of a polyester sheath-core type were mixed so as to prepare a card web having a weight (Metsuke) of 30g/m² by using a roller card. The difference of MD/CD of the web was approximately MD/CD=3.5 by strength ratio. This web was introduced onto a net conveyer which was provided with two types high pressure water-jets, and then subjected to a water entanglement at a speed of about 30m/min. on a net which was provided with a dewatering zone. The conditions of the water entanglement were as follows:

| First Treating Zone | |
|---|---|
| Number of Nozzle Lines | 2 sets |
| Constitutive Nozzle | Diameter: 0.15mm |
| | Spacing : 0.6 mm |
| Water Pressure | 50kg/cm² |

| Second Treating Zone | |
|---|---|
| Number of Nozzle Lines | 2 sets |
| Constitutive Nozzle | Diameter: 0.20mm |
| | Spacing : 1.00mm |
| Water Pressure | 70kg/cm² |

The web after water entangling was dewatered, and introduced into a hot-air dryer so as to dry. The maximum temperature was 130O°C. After passing the dryer, the web was drawn by approximately 30% (which corresponds to 1.3 times) in a heated state, and then a cold winding was carried out.

The non-woven fabric obtained thereby had a weight(Metsuke) of 22g/m² and a directional difference of MD/CD=8. A elongation percentage after breaking in the CD was about 280%.

### 〈Manufacture of Elastic Body Sheet〉

### Preparation of Compounds

65 parts of S.E.B.S. resin (the trade name "SEPTON #8007" made by KURARAY, Co., LTD.); 35 parts of E.V.A. resin (the trade name "EVA FLEX P-1907" made by Mitsui DuPont Ltd.); and 0.1 parts of an oxidation inhibitor (the trade name "RUGANOX 1010" made by Mitsui DuPont Ltd.) were added and mixed, and melted-pelletized so as to prepare a compound. The MFR(g/10min.) of the compound was 7.8 at a temperature of 230O°C and a pressure of 2.16kg.

### 〈Forming and Winding of Elastic Film〉

A film having a thickness of 20µm was formed from the compound by using a die-extruding machine. Since this film has a high self adhesion property, if it is wound as it is, it adhere closely, it comes to be impossible to separate it. Therefore, the non-woven fabric was inserted before winding the film, and the film was laminated to the non-woven fabric so as to wind them.

### 〈Manufacture of Jointed Body in Which Nonwoven Fabric Is Jointed at One Side〉

The sheet which were wound by laminating the non-woven fabric over the elastic film is temporarily jointed owing to the self adhesion of the film. This sheet which is temporarily jointed was introduced onto a 40-mesh plastic net so as to layer in the order of the net, the elastic film and the non-woven fabric, which were then passed through a pressure apparatus which comprises a combination of a heated grid roller/a flat roller, so that the elastic film and the non-woven fabric having an elongation property are partially welded. The heating was carried out at the non-woven fabric side. The outline of the heating-pressure apparatus is as follows:

| Upper Roller (Grid Roller) | |
|---|---|
| Height of Thread | 1.0mm |
| Width of Top | 1.5mm |
| Grid Spacing | 3.0mm |
| Inner Heat Medium Heating | |
| Process Temperature | 130O°C |
| Surface Treatment | Chrome Plating |

| Lower Roller (Flat Roller) | |
|---|---|
| Surface Treatment | Chrome Plating |
| Process Temperature | 130O°C |
| Pressure | 40kg/cm² |
| Winding Speed | 20m/min. |

According to such procedure, a composite elastic material which is a jointed body of the film and the non-woven fabric was obtained. A joint pattern of the composite elastic material had a pattern as shown in Figure 25.

### 〈Manufacture of Jointed Body in Which Nonwoven Fabrics Are Jointed at Both Sides〉

Two sheets of the one-side jointed-body were bonded to each other so that the sides with the film can face each other, so as to prepare a composite elastic material having a non-woven fabric at each side thereof.

In this example, since an self adhesion property between the films is very large, the films are easily jointed to each other by laminating the films to each other and thermo-compressing the same. In this example, when the two sheets were passed through two flat rollers which were heated to 80O°C, a composite elastic material was obtained, which has a joint structure as shown in Figure 19 and has non-woven fabrics on both sides by which the two sheets were jointed to each other and united.

### 〈Determination of S-S Curve of Composite Elastic Body〉

By using the composite elastic material, an S-S curve which results in a complete breaking was determined. Figure 2 shows the determined result. Both a stress-lowering point at approximately 230% on the basis of breaking of the sections of the non-woven fabrics, and a stress-lowering point at approximately 420% on the basis of breaking of the films are observed in Figure 2 also.

### 〈Determination of S-S Curve of Drawn Article〉

By using the composite elastic material, three types samples were prepared, each of which were predrawn by 75%, 100% and 150%, respectively. A S-S curve of each sample, which results in complete breaking, were determined. The results are shown in Figures 3 to 5. In any cases, the existence of a first stress-lowering point and a second stress-lowering point comes to be more clearly observed.

It is supposed that a strain was removed by drawing, and thereby the homogenization of the structure was caused, and therefore the results were obtained.

It is further important that such drawing makes an elongating stress within the drawing sharply lower. This is an important point of the present invention, which is based on the elongation activation. A composite elastic material which was drawn and worked with such effects comes to have four areas having elongation properties, as shown in Figure 6.

### (Example 2)

### 〈Manufacture of Nonwoven Fabric Having Elongation Properties〉

50 parts of a polyester fiber (1/5d × 35mm) and 50 parts of a polyester fiber (2d × 51mm) were mixed so as to prepare a parallel card web of 25g/m² by using a roller card.

The difference between the MD and CD strength of the web was MD/CD=7. This web was introduced onto a multiple aperture suction cylinder which was provided with three nozzles and a dewatering zone, and after water saturation, deaeration and dewater, passed through the nozzles at a velocity of 30m/min. so as to carry out a water entanglement:
- First Nozzle: 0.12mm⌀ × 0.4mm Spacing
Water Pressure 30kg/cm²
- Second Nozzle: 0.12mm⌀ × 0.4mm Spacing
Water Pressure 50kg/cm²
- Third Nozzle: 0.20mm⌀ × 1.5mm Spacing
Water Pressure 60kg/cm²

The above-mentioned web by the water entanglement was dried and heated so as to obtain a web of non-woven fabric.

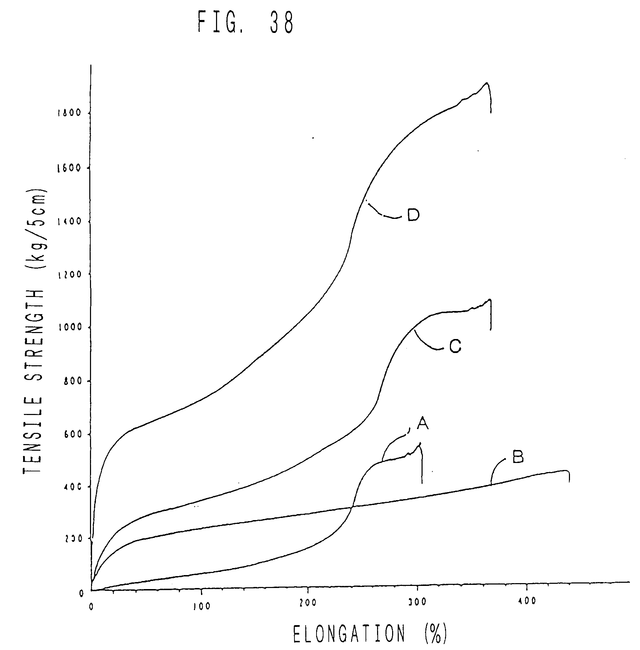

A S-S curve of the non-woven fabric until a first stress-lowering point in the CD thereof was as shown in Figure 38(A).

### 〈Preparation of Elastic Body Sheet〉

A blended resin comprising a polyolefin elastomer of EMA/EPDM was extruded so as to prepare a film having a thickness of 25µm as an elastic sheet. A S-S curve in the CD of the elastic sheet was as shown in Figure 38(B).

### 〈One Side Jointed Body〉

The above-mentioned non-woven fabric and elastic sheet were laminated to each other, and placed on a 60-mesh PFT net so that the elastic sheet is on the side of the PFT net, and then thermo-compressed at a linear load of 10kg/cm so that a heated roller at a temperature of 110O°C, which has many embossed patterns, comes in contact with the non-woven fabric, while a flat roller comes in contact with the net, so as to obtain a composite elastic material.

A S-S curve until a first stress-lowering point in the CD of the composite elastic material was as shown in Figure 38(C).

Furthermore, the result of a three-cycle test in which a set of elongating the composite elastic material to 150% and easing it was repeated is as shown in Figure 7, in which the composite elastic material had a recovery percentage of 75%.

### 〈Both Sides Jointed Body〉

Two sheets of the above-mentioned one side jointed body were laminated to each other so that the film sides can face each other, and passed through a heated roller having a flat surface at a temperature of 80O°C with a linear load of 20kg/cm at a velocity of 10m/min. so as to compress them, and then were in a stable jointed state at the film sides, in which the positions of joint points were shifted so that the inside does not overlap to the outside. A S-S curve until a first stress-lowering point in the CD of the composite elastic material was as shown in Figure 38(D).

Furthermore, the result of a three-cycle test in which a set of elongating the composite elastic material to 150% and easing it was repeated is as shown in Figure 8, in which the composite elastic material had a recovery percentage of 75%.

### (Example 3)

### 〈One Side Jointed Body of S.E.B.S. Film and Nonwoven Fabric〉

A composition was extruded so as to prepare an elastic film having a thickness of 25µm; the composition comprising a resin as a main component, which was blended by mixing 75 parts with S.E.B.S. and 25 parts of E.V.A. This film had a property that the film easily adheres to itself merely by crimping it at an ordinary temperature.

A rubber hot melt adhesive was sprayed in a very small amount (approximately 0.4g/m²) on one side of the film, and jointed to a non-woven fabric which is similar to the one used in Example 1, by crimping all over the surface.

### 〈Both Sides Jointed Body〉

Two sheets of composite material, in each of which a non-woven fabric is jointed to one side of the above-mentioned S.E.B.S. film, were prepared, and laminated to each other so that the films having an self adhesive property can face each other, passed between a pair of flat rollers at a linear load of 20kg/cm² at a temperature of about 40O°C so as to obtain a jointed body in which the films were stably jointed to each other, and the non-woven fabrics were jointed at both sides thereof.

The one-side jointed body and the both-sides jointed body which are constituted as mentioned above had an elongation recovering property which is similar to the one in Example 1, respectively.

### (Example 4)

### Preparation of Nonwoven Fabric as Raw Material

A spun-bond non-woven fabric (trade name "ELVES" made by UNITIKA, LTD.) was prepared, which comprises a conjugated fiber having a polyester core as a skeleton component(B) and a polyester sheath as a jointing component(A), and has a width of 1m and a weight of 25g/m². This non-woven fabric is the one which was manufactured by forming a web according to spun-bond process, and then distributing spot bond-points with a predetermined density. The percentage of the distributed spot bond-points was approximately 8% as a ratio thereof to the whole area, and the physical properties were as follows. A plasticizing temperature of PET as a skeleton component(B) was about 190O°C, and a stable temperature area was in the range of about 1000 to about 130O°C.

| Tensile Strength | |
|---|---|
| Machine Direction | 12.5kgf/5cm |
| Cross Direction | 4.5kgf/5cm |
| Ratio of Machine/Cross | 2.8 |

| Elongation Percentage after Fracture | |
|---|---|
| Machine Direction | 60% |
| Cross Direction | 60% |
| Ratio of Machine/Cross | 1 |

The above-mentioned spun-bond non-woven fabric was introduced into a pressure steaming machine which is provided with a clip tenter at a speed of about 10m/min., and heated at a temperature of 105O°C to 115O°C in the steaming machine while widen to about 1.5 times. The non-woven fabric ejected from the steaming machine was dried at an ordinary temperature, and wound. Some water was left in the widened non-woven fabric. The weight thereof was about 18g/m². Then, the widened non-woven fabric was passed through a multiple aperture cylinder which was provided with a steam generator so as to draw it to about 2.2 times the natural length thereof in a machine direction (MD), dried in hot air at a temperature of 60O°C, and wound. The weight thereof was about 22g/m².

The resultant easily stretchable non-woven fabric had mesh apertures and softness, and was very rich in an stretchable property in a cross direction.

The physical properties were as follows:

| Tensile Strength | |
|---|---|
| Machine Direction | 8.7kgf/5cm |
| Cross Direction | 1.5kgf/5cm |
| Ratio of Machine/Cross | 5.8 |

| Elongation Percentage after Fracture | |
|---|---|
| Machine Direction | 30% |
| Cross Direction | 280% |
| Ratio of Machine/Cross | 9.3 |

This result shows that as compared with the non-woven fabric of the raw material, the resultant non-woven fabric are sharply elevated in an elongation percentage in a cross direction (which means an perpendicular direction to the drawing direction).

### 〈Manufacture of Elastic Body Compound〉

45 parts of S.E.P.S. (trade name "SEPTON #4033" made by KURARAY CO., LTD.), 30 parts of LDPE(trade name "LM31" made by UNITIKA, LTD.) and 25 parts of a processed oil(trade name "DIANA PW-380" made by UNITIKA, LTD.) were mixed so as to prepare a pelletized compound. An MFR(g/10min.) of the compound was 14 at a temperature under a pressure of 2.1kg.

### 〈Formation of Elastic Film & Jointing of Nonwoven Fabric Having Elongation Property〉

By using the above-mentioned compound, a film having a thickness of 30µm through a die forming machine. Before the film was cooled, it was laminated to the above-mentioned spun-bond having an elongation property, passed through a pair of flat press rollers, and thereafter further passed through a pair of heat embossed rollers.

The heat embossing was carried out at the side of the non-woven fabric.

| Upper Roller (Embossed Projecting Roller) | |
|---|---|
| Height of Thread | 0.8mm |
| Pattern | Pattern as Shown in Figure 27 |
| Grid Spacing | 3.0mm |
| Inner Heat Medium Heating | |
| Process Temperature | 120O°C |
| Surface Treatment | Chrome Plating |

| Lower Roller (Flat Roller) | |
|---|---|
| Surface Treatment | Chrome Plating |
| Process Temperature | Ordinary Temperature |
| Pressure | 30kg/cm² |

Thus, a composite elastic material comprising a film and a non-woven fabric having an elongation property was obtained according to an on-line processing. The resultant composite elastic material showed an excellent elastic property which is similar to the ones in each of the above-mentioned Examples.

### (Example 5)

A non-bonded web of PET spun-bond having a weight of about 10g/m² was prepared as a skeleton component (B), and laminated to a melt-blown web having PE of a weight of about 7g/m² as a main component, and then subjected to a mesh bonding so as to prepare a double-layer non-woven fabric of about 24g/m² having a constitution as shown in Figure 16. This double-layer non-woven fabric was drawn to about 1.8 times the natural length thereof, while heated by multistage rollers of a drawing apparatus which was provided with infrared lamps in the upper and the lower sides at a temperature of about 120O°C according to a continuous process.

Thereby, an easily stretchable non-woven fabric was prepared, which is soft and excellent in an stretchable property in a cross direction.

The physical properties were as follows:

| Tensile Strength | |
|---|---|
| Machine Direction | 9.5 kgf/5cm |
| Cross Direction | 0.85kgf/5cm |
| Ratio of Machine/Cross | 11.0 |

| Elongation Percentage after Fracture | |
|---|---|
| Machine Direction | 30% |
| Cross Direction | 830% |
| Ratio of Machine/Cross | 6 |

The non-woven fabrics were laminated onto both sides of an elastomer film so that the elastomer film is sandwiched between the non-woven fabrics, and laminated to a 40-mesh plastic net, and then passed between a pair of flat rollers under a pressure of 2kg/cm² so as to obtain a composite elastic material with a point-joint; the elastomer film having S.E.B.S. as a main component and a thickness of 40µm, and being made by U.S.A. KUROPEI LTD.

The present composite elastic material is excellent in an elastic property, and has a first breaking point based on breaking of the non-woven fabrics and a second breaking point based on breaking of the elastic material. The properties were as follows:

| | |
|---|---|
| Residual Strain | 15% |
| First Stage Elongation after Fracture | 185% |
| Second Stage Elongation after Fracture | 385% |

### (Example 6)

Burst fiber webs of polypropylene were laminated to both sides of a widened web of polyester tows having a weight of 30g/m² so that the widened web is sandwiched between the burst fiber webs, so as to prepare a non-woven fabric (the trade name "UNICEL" made by TEIJIN LTD.). The structure was as shown in Figure 17, and the physical properties were as follows:

| Tensile Strength | |
|---|---|
| Machine Direction | 6.3kgf/5cm |
| Cross Direction | 6.5kgf/5cm |
| Ratio of Machine/Cross | 1.0 |

| Elongation Percentage after Fracture | |
|---|---|
| Machine Direction | 80% |
| Cross Direction | 60% |
| Ratio of Machine/Cross | 0.8 |

### Heating Conditions

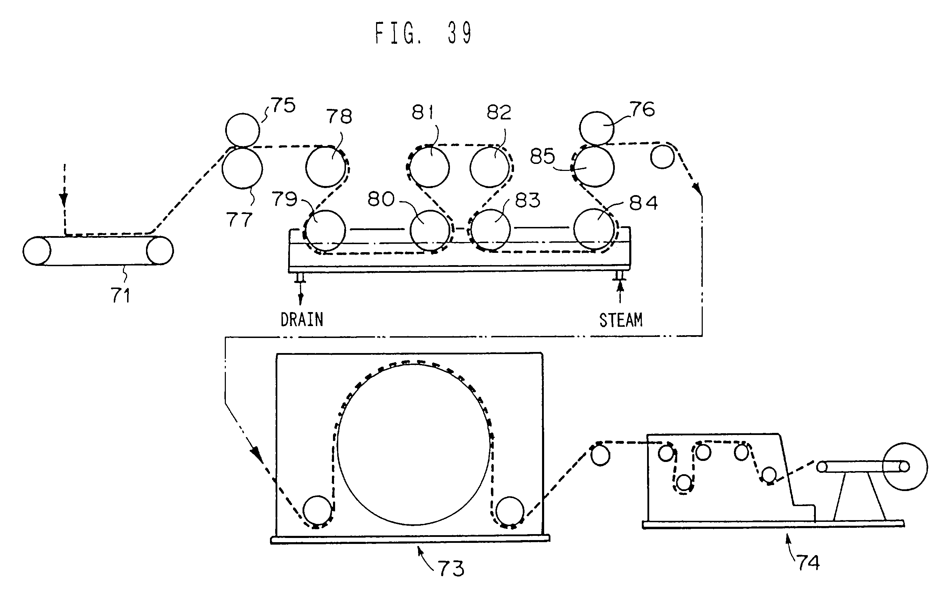

The above-mentioned non-woven fabric having a width of 1m was treated by using a thermal pretreatment equipment as shown in Figure 39. This equipment were provided with belt conveyer (71), a drawing machine (72), a dryer (73) and a winder (74). The above-mentioned non-woven fabric was first fed from the conveyer (71) to the drawing machine (72) which comprises two press rollers (75, 76) and the other nine rollers (77 to 85) which were arranged by two stages; the roller (77) facing the press roller (75), the roller (85) facing the press roller (76), the roller (79, 80, 83 and 84) which are positioned at the lower stage being dipped at the lower end of each in hot water with which a hot-water bath (86) is stored. Steam is introduced in hot water in the hot-water bath (86) so as to maintain a temperature of the boiling point of hot water of or a near point thereof.

The non-woven fabric ejected from the conveyer (71) was passed between the rollers (75) and (77), and passed over the roller (78) which acts as a widening roller, and then passed over rollers (79-84) in order, and finally passed between the press roller (76) and the roller (85) so as to be drawn out. By passing over each roller, the non-woven fabric is by stages drawn while heated. The drawn non-woven fabric is introduced into the dryer (73) to be dried in hot air, and wound by the winder (74).

In this Example, the non-woven fabric was by stages drawn to about 2.0 times the natural length thereof while passed through the drawing machine (72) at a speed of about 20m/min., and dried in a hot air at a temperature of about 70O°C in the dryer (73), and then wound.

The resultant non-woven fabric had some splinters, while had a soft and large stretchable property in a weft direction. A weight was about 20kg/m².

### Easily Extensible Nonwoven Fabric

The resultant non-woven fabric had a lacelike appearance, and was remarkably excellent in an stretchable property in a weft direction.

The physical properties were as follows:

| Tensile Strength | |
|---|---|
| Machine Direction | 4.0kgf/5cm |
| Cross Direction | 1.1kgf/5cm |
| Ratio of Machine/Cross | 3.6 |

| Elongation Percentage after Fracture | |
|---|---|
| Machine Direction | 42% |
| Cross Direction | 258% |
| Ratio of Machine/Cross | 6.1 |

It is found that the elongation percentage in a cross direction is sharply elevated as compared with the raw material before drawing.

This non-woven fabrics having an easily stretchable property were laminated to both sides of a netty S.E.B.S. elastic material (the trade name "NETRON" made by Mitsui Petrochemical Industries, Ltd) having a weight of 110g/cm², and wholly thermo-jointed with spots. The resultant non-woven fabrics having an easily stretchable property were rich in an elastic property, and had a first breaking point based on breaking of the non-woven fabrics, and a second breaking point based on breaking of breaking of the elastic material.

A first-step elongation percentage after breaking was 320%, and a second-step elongation percentage after breaking was 440%.

### (Example 7)

### Preparation of Easily Extensible Nonwoven Fabric in Cross Direction

A spun-bond non-woven fabric (the trade name "ELVES" made by UNITIKA, LTD.) having a weight of 25g/m² was drawn to about two times in a machine direction according to a process as shown in Figure 39 so as to obtain a non-woven fabric having an easily stretchable property, a weight of 18g/m², and the following performance; the spun-bond non-woven fabric comprising a conjugated a fiber which has a polyester core and a polyethylene sheath at the ratio of the former to the latter of 50/50.

| Tensile Strength | |
|---|---|
| Machine Direction | 6.8kgf/5cm |
| Cross Direction | 1.4kgf/5cm |
| Ratio of Machine/Cross | 5.0 |

| Elongation Percentage after Fracture | |
|---|---|
| Machine Direction | 40% |
| Cross Direction | 220% |
| Ratio of Machine/Cross | 5.5 |

### Preparation of Elastic Sheet

A resin (Ra) having S.E.B.S. as a main component and a blended resin (Rb) of S.E.B.S. of 60%/ E.V.A. of 40% were used so as to prepare a coextruded film according to a process as shown in Figure 23. The resultant film had a thickness of 50µm (Ra layer: 35µm, Rb layer: 15µm). The physical properties of the film were determined, and the following values were obtained:

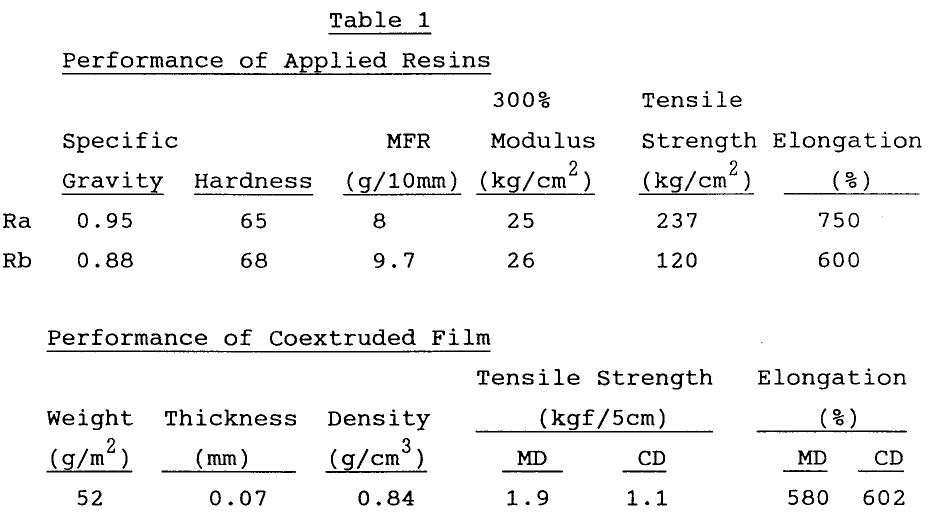

### Joint and Easement of Elastic Body Sheet and Nonwoven Fabric Having Easily Extensible Property

According to a process as shown in Figure 40, the above-mentioned elastic material sheet was drawn to 2.5 times the natural length thereof in a machine direction, and the above-mentioned non-woven fabric having an easily stretchable property was laminated onto the surface of the Ra layer at the MD which is excellent in a dimensional stability, while the elastic sheet was maintained in a drawn state. Then, the elastic sheet was passed between a chrome plated roller and a silicone rubber roller at a linear pressure of 10kg/cm, so as to joint the elastic sheet to the non-woven fabric; the rollers having an emboss pattern as shown in Figure 27, the surfaces of the rollers being heated at a temperature of 120O °C. Incidentally, the heating was carried out at the side of the non-woven fabric.

After jointing, the elastic sheet and the non-woven fabric were restored to an easy state, and the elastic sheet was eased so as to obtain a composite elastic material which has a two-layer structure having creepy corrugations in a machine direction.

This composite elastic material was easily elongated in a machine direction, while showed a little resistance against elongating in a cross direction. Furthermore, when the composite elastic material was previously predrawn by about 150% in a cross direction, it came very stretchable in both machine and cross directions. The elastic properties are shown as follows:

**Table 2**

| Performance of Two-Layer Composite Elastic Material | | | | | | |
|---|---|---|---|---|---|---|
| | Tensile Strength (kgf/5cm) | Elongation (%) | Elasticity (g/5cm) | | | Residual Strain (%) |
| | | | 50% | 100% | 150% | |
| MD | 7.5 | 275 | 90 | 95 | 105 | 15 |
| CD | 1.8 | 400 | 120 | 160 | 180 | 22 |
| | | (First Stage 230) | | | | |

When the two-layer composite elastic material was elongated in a machine direction, the non-woven fabric was fractured, and approximately simultaneously the elastic material was broken, while the composite elastic material had a first stress-lowering point and a second stress-lowering point that the non-woven fabric was fractured at an about 230% point in a cross direction, and then only the film was elongated, the film was fractured at an about 230% point.

As mentioned above, while a composite elastic material of the present invention is elongated from a non-elongated state until reach to the breaking limit, stress i.e., resistance to an elongation is gradually increased as it approaches the breaking limit, and the stress will approach the maximum just before the breaking limit. Then, when the composite elastic material is further elongated, the stress reaches a first stress-lowering point which is caused by breaking of a non-woven fabric, and is rapidly lowered, and thereafter, the composite elastic material is elongated with a small stress until the breaking limit of the elastic material. Moreover, even if the composite elastic material is elongated until any stages, if the tensile force is eased, it can be restored to the original length due to the elastic property of the elastic material. Due to such elongation properties, the composite elastic material of the present invention is excellent in a stretch-recovery property, and the touch of the surface thereof. Thus, the composite elastic material can be advantageously used for the purpose of, in particular, an elastic material applied to parts which directly touch the skin, such as an elastic material which is provided for the sleeve portion of a medical gown, or the waist or crotch region of sanitary articles.

## Claims

1. A composite elastic material having a multiple-stage elongation property comprising:
a non-woven fabric which is stretchable along at least one direction and has a break-down elasticity along said direction of 100% or more; and
a sheet-like elastic member having an elastic recovery of 60% or more and a break-down elasticity of 200% or more;
said non-woven fabric and said elastic member being secured together at a plurality of securements, so that said composite elastic material has a first stress lowering point caused by changes of structure of said non-woven fabric while being stretched along said direction, and a second stress lowering point occurring at an elongation larger than that said first stress lowering point lies, caused by breakdown of said sheet-like elastic member.

2. The composite elastic material claimed in claim 1, wherein the breakdown elasticity of said non-woven fabric is 150% or more, and the break-down elasticity of said elastic member is 250% or more, and the difference in the break-down elasticity between said non-woven fabric and said elastic member is 100% or more.

3. The composite elastic material claimed in claim 1 or 2, wherein the difference in elongation between said first and second stress lowering points is 50% or more.

4. The composite elastic material claimed in any one of claims 1-3, wherein the break-down elasticity of said non-woven fabric is 150% or more, preferably 200% or more.

5. The composite elastic material claimed in any one of claims 1-4, which is stretchable in both the longitudinal and transverse directions, and has said first and second stress lowering points while being stretched along the longitudinal or transverse direction thereof.

6. The composite elastic material claimed in any one of claims 1-5, wherein said non-woven fabric is a stretch-paralleled spun-bond non-woven fabric or a stretch-paralleled melt-blown non-woven fabric.

7. The composite elastic material claimed in any one of claims 1-5, wherein said non-woven fabric is manufactured through entanglement by water stream and has a dual stage elongation of different stress levels.

8. The composite elastic material claimed in claim 7, wherein the second stage elongation of said non-woven fabric occurs when stretched over 150% or more.

9. The composite elastic material claimed in any one of claims 1-8, wherein said non-woven fabric has an increased elongation in the cross-machine direction due to its high degree orientation of fibers of said non-woven fabric by hot stretching along the machine direction.

10. The composite elastic material claimed in any one of claims 1-9, wherein said non-woven fabric comprises an easily fusible material.

11. The composite elastic material claimed in claim 1, wherein said non-woven fabric ia an easily stretchable composite non-woven fabric comprising a fibrous material which comprises a bonding component (A) of thermoplastic property and a skeleton component (B) having a higher stability against heat than that of said component (A), said composite non-woven fabric being manufactured by stretching in one direction under heat at a temperature of above the plasticizing temperature of said bonding component (A) and in the range of stable temperature of said skeleton component (B), whereby having an elongation of 100% or more in the direction perpendicular to said stretching direction.

12. The composite elastic material claimed in claim 11, wherein said composite non-woven fabric is mainly consisting of a highly random filament non-woven fabric of which the ratio of strength of longitudinal/transverse ratio is below 3.0.

13. The composite elastic material claimed in claim 11, wherein said composite non-woven fabric comprises as a main component a spun-bond comprised of conjugate fibers each consisting of a sheath of polyethylene and a core of polyester, and the content of polyethylene being 40% or over.

14. The composite elastic material claimed in any one of claims 11-13, wherein said non-woven fabric comprises a core layer of unbonded spun-bond and one or two melt-blown web of thermoplastic resin disposed upper and/or lower surface of said core layer.

15. The composite elastic material claimed in any one of claims 11-14, wherein said composite non-woven fabric comprises a layer of unbonded filaments which have been opened and spreaded, and a layer or layers of fibril thermoplastic resin laminated on the upper and/or lower surfaces.

16. The composite elastic material claimed in any one of claims 11-15, wherein said sheet-like elastic member is composed of an easily fusible material.

17. The composite elastic material claimed in any one of claims 1-15, wherein said sheet-like elastic member consists of a foam of urethane or rubber latex; a synthetic rubber film of isoprene or butadiene; a styrene-series elastomer film of SIS, SEBS, SEPS or the like; a polyolefin-series elastomer film of EVA, EMA, EPDM or the like; or a melt-brown elastomer non-woven fabric of polyurethane, SIS, SEBS or the like.

18. The composite elastic material claimed in any one of claims 1-17, wherein said non-woven fabrics are disposed on and secured to both sides of said sheet-like elastic member.

19. The composite elastic material claimed in any one of claims 1-17, which comprises two elastic members each of which is secured to said non-woven fabric on its surface, said elastic members facing and being secured together to form a four layers construction.

20. The composite elastic material claimed in claim 18 or 19, wherein one of said non-woven fabric is hydrophilic and the other is hydrophobic.

21. The composite elastic material claimed in any one of claims 1-20, wherein said non-woven fabric and said elastic member is secured at spot securements disposed in random.

22. The composite elastic material claimed in any one of claims 18-21, wherein a first non-woven fabric is disposed on one surface of said elastic member and a second non-woven fabric is disposed on said elastic member, said first and said non-woven fabric being secured to said elastic member at a plurality of spot securements, and said securements between said first non-woven fabric and said elastic member being disposed in a manner to not overlap with said securements between said second non-woven fabric and said elastic member.

23. The composite elastic material claimed in any one of claims 1-20, wherein each of said securements connecting said sheet-like elastic member and said non-woven fabric extends in a direction perpendicular to the elongation direction of said non-woven fabric in the form of a belt.

24. The composite elastic material claimed in any one of claims 1-20, wherein said securements connecting said sheet-like elastic member and said non-woven fabric are disposed in a plurality of rows extending in a direction perpendicular to the elongation direction of said non-woven fabric.

25. The composite elastic material claimed in claim 23 or 24, which is caused to be stretchable in a specific direction by heating and compressing at least one portion at a temperature higher than the melting temperature of said sheet-like elastic member and lower than the melting temperature of said non-woven fabric, thereby forming at least one portion having smaller elongation than that of the remaining portion.

26. The composite elastic material claimed in claim 25, wherein each of said portion having smaller elongation is formed in a strip.

27. The composite elastic material claimed in any one of claims 1-26, which comprises one or more portions having higher elongation at which said sheet-like elastic member and said non-woven fabric are secured at selected securement portions and one or more portions having lower elongation, compared to that of said selected securement portions, at which said sheet-like elastic member and said non-woven fabric are secured at their whole surfaces.

28. The composite elastic material claimed in claim 27, wherein said portions having smaller elongation are formed at strip-like securements disposed in parallel.

29. The composite elastic material claimed in claim 27, wherein a pair of portions having smaller elongation at both sides of said elastic material.

30. An absorbent article which has a form capable of covering the loins of the wearer and comprises an absorbent material, comprising the composite elastic material claimed in claim 29 is connected at said portion having smaller elongation formed at one end thereof to a main body of said absorbent article, and connected at the other end to a fastener.

31. An absorbent article which has a form capable of covering the loins of the wearer and comprises an absorbent material, comprising side panels formed of said composite elastic material claimed in claim 29.

32. A method of manufacturing composite elastic material having a multiple-stage elongation comprising the steps of:
lying a non-woven fabric having an elongation along at least one direction and a break-down elasticity along said direction of 100% or more on a sheet-like elastic member having an elastic recovery of 60% or more and a break-down elasticity of 200% or more;
securing said non-woven fabric and said elastic member together at a plurality of securements discontinuous in the stretchable direction of said non-woven fabric to form a composite material; and
conducting previous elongation to said composite material in an stretchable direction of said non-woven fabric with an elongation degree smaller than the break-down elasticity of said elastic member to have a first stress lowering point caused by changes of structure of said non-woven fabric and a second stress lowering point occurring at an elongation larger than that said first stress lowering point lies, caused by break-down of said sheet-like elastic member.

33. The method claimed in claim 32, wherein said previous elongation is conducted at an elongation of 40%-80% of the break-down elasticity of said non-woven fabric.

34. A method of manufacturing composite elastic material comprising the steps of:
providing a first sheet-like elastic member by extruding a resin having a self-adhering property;
providing a first composite sheet by continuously supplying a non-woven fabric on said first elastic member and by thermally securing together at a desired pattern;
providing a second sheet-like elastic member by extruding a resin having a self-adhering property;
providing a second composite sheet by continuously supplying a non-woven fabric on said second elastic member and by thermally securing together at a desired pattern; and
introducing said first and second composite sheets with facing said first sheet-like elastic member to said second sheet-like elastic member thereby adhering together by their self-adhesion property.

35. A method of manufacturing composite elastic material comprising the steps of:
providing a sheet-like elastic member by coextruding a first resin having a self-adhering property and a second resin having a less self-adhering property to form a first layer of higher self-adhering property and a second layer of lower self-adhering property;
continuously supplying a non-woven fabric on said second layer of said sheet-like elastic member; and
thermally securing said sheet-like elastic member to said non-woven fabric with securements having a desired pattern.

36. A method of manufacturing composite elastic material having elasticity in both the longitudinal and transverse directions, comprising the steps of:
elongating a sheet-like elastic member which has an elastic recovery of 60% or more and a breakdown elasticity of 200% or more, in a longitudinal direction at 100% or more;
lying and securing a non-woven fabric which has a break-down elasticity in longitudinal direction of 50% or less and in transverse direction of 100% or more, while said sheet-like elastic member is kept stretched;
releasing the stretched condition of said sheet-like elastic member.
